# EUROPÄISCHE PATENTSCHRIFT

(11) **EP 2 730 332 B1**
(45) Veröffentlichungstag und Bekanntmachung des Hinweises auf die Patenterteilung: **31.07.2019**
(21) Anmeldenummer: 13189552.6
(22) Anmeldetag: 21.10.2013
(51) Int. Cl.: B01F 11/00, B01F 13/00, B01F 15/00, B01F 15/02

(54) **Vorrichtung zum Mischen und Austragen einer pastösen Masse**
Device for mixing and applying a paste-like mass
Dispositif de mélange et d'extraction d'une masse pâteuse

(30) Priorität: 07.11.2012 DE 102012021676; 18.12.2012 DE 102012024710
(43) Veröffentlichungstag der Anmeldung: 14.05.2014
(73) Patentinhaber: Heraeus Medical GmbH, 61273 Wehrheim (DE)
(72) Erfinder: Vogt, Sebastian, 99092 Erfurt (DE)
(74) Vertreter: Schultheiss & Sterzel Patentanwälte PartG mbB

(56) Entgegenhaltungen:
- WO-A1-2009/105905

## Beschreibung

Die Erfindung betrifft eine Vorrichtung zum Mischen und Austragen einer pastösen Masse gemäß dem Oberbegriff des Anspruchs 1, insbesondere eines Knochenzements. Ferner betrifft die Erfindung ein Knochenzementsystem aufweisend eine solche Vorrichtung und ein Verfahren zum Herstellen eines Zementgemischs, insbesondere eines Knochenzements mit einer solchen Vorrichtung oder einem solchen Knochenzementsystem.

Polymethylmethacrylat-(PMMA)-Knochenzemente gehen auf die grundlegenden Arbeiten von Sir Charnley zurück. PMMA-Knochenzemente bestehen aus einer flüssigen Monomerkomponente und einer Pulverkomponente. Die Monomerkomponente enthält im Allgemeinen das Monomer Methylmethacrylat und einen darin gelösten Aktivator (N,N-Dimethyl-p-toluidin). Die Pulverkomponente, auch als Knochenzementpulver bezeichnet, weist ein oder mehrere Polymere, die auf Basis von Methylmethacrylat und Comonomeren, wie Styren, Methylacrylat oder ähnlichen Monomeren durch Polymerisation, vorzugsweise Suspensionspolymerisation, hergestellt sind, einen Röntgenopaker und den Initiator Dibenzoylperoxid auf. Beim Vermischen der Pulverkomponente mit der Monomerkomponente entsteht durch Quellung der Polymere der Pulverkomponente im Methylmethacrylat ein plastisch verformbarer Teig, der eigentliche Knochenzement. Beim Vermischen der Pulverkomponente mit der Monomerkomponente reagiert der Aktivator N,N-Dimethyl-p-toluidin mit Dibenzoylperoxid unter Bildung von Radikalen. Die gebildeten Radikale initiieren die radikalische Polymerisation des Methylmethacrylates. Mit fortschreitender Polymerisation des Methylmethacrylates erhöht sich die Viskosität des Zementteigs, bis dieser erstarrt.

Polymethylmethacrylat-Knochenzemente können in geeigneten Mischbechern mit Hilfe von Spateln durch Vermischen des Zementpulvers mit der Monomerflüssigkeit vermischt werden. Nachteilig ist an dieser Vorgehensweise, dass Lufteinschlüsse im gebildeten Zementteig vorhanden sein können, die später eine Destabilisierung des Knochenzements verursachen können. Aus diesem Grund wird das Vermischen von Knochenzementpulver mit der Monomerflüssigkeit in Vakuummischsystemen bevorzugt, weil durch Mischen im Vakuum Lufteinschlüsse aus dem Zementteig weitgehend entfernt werden und damit eine optimale Zementqualität erreicht wird. Im Vakuum gemischte Knochenzemente haben eine deutlich verringerte Porosität und zeigen daher verbesserte mechanische Eigenschaften. Es wurden eine Vielzahl von Vakuum-Zementiersystemen offen gelegt, von denen exemplarisch folgende genannt sind: US 6,033,105 A, US 5,624,184 A, US 4,671,263 A, US 4,973,168 A, US 5,100,241 A, WO 99/67015 A1, EP 1 020 167 A2, US 5,586,821 A, EP 1 016 452 A2, DE 36 40 279 A1, WO 94/26403 A1, EP 1 005 901 A2, US 5,344,232 A.

Eine Weiterentwicklung stellen Zementiersysteme dar, in denen sowohl das Zementpulver als auch die Monomerflüssigkeit bereits in separaten Kompartimenten der Mischsysteme verpackt sind und erst unmittelbar vor der Zementapplikation im Zementiersystem miteinander vermischt werden, wie beispielsweise mit der EP 0 692 229 A1 vorgeschlagen. Ein wesentliches Problem dieser Systeme ist die Sterilisation des gesamten Systems einschließlich des Zementpulvers und der zuvor steril filtrierten Monomerflüssigkeit. Problematisch ist insbesondere die Durchführung der bei Knochenzementen häufig genutzten Sterilisation mit Ethylenoxid. Diese Sterilisationsmethode hat gegenüber der Sterilisation mit Gammastrahlen den Vorteil, dass die im Zementpulver enthaltenen Polymere nicht degradiert werden und die Zementeigenschaften durch die Ethylenoxid-Sterilisation unbeeinflusst bleiben. Problematisch ist bei der Sterilisation mit Ethylenoxid, dass das gasförmige Agens zuerst in die Kartusche bzw. den Zementvorratsbehälter und damit in das Zementpulver eindringen muss und nach der Sterilisation wieder aus der Kartusche heraus diffundieren muss. Ein möglichst ungehinderter Gasaustausch zwischen dem Inneren der Kartusche beziehungsweise des Vorratsbehälters und der Umgebung ist daher zwingend erforderlich. Im Widerspruch dazu muss das einsatzbereite Mischsystem so dicht verschlossen sein, dass ein Anmischen des Zements unter Vakuum erfolgen kann.

Bei auf dem Markt befindlichen Mischsystemen wird dieser Widerspruch dadurch gelöst, dass auf der Kartusche ein Deckel mit einer porösen Scheibe aufgeschraubt ist, der unmittelbar vor der Zementapplikation entfernt werden muss. Anstelle dieses Deckels wird ein vakuumdichter Kartuschenkopf aufgeschraubt, der eine Mischvorrichtung, einen Vakuumanschluss und eine Öffnung für das später aufzusetzende Austragsrohr enthält.

Der medizinische Anwender muss daher das Zementiersystem unmittelbar vor der Zementanmischung öffnen und dann wieder verschließen. Dadurch können Keime etc. an das zuvor sterilisierte Knochenzementpulver gelangen.

Mit der DE 10 2009 031 178 B3 wird eine Vorrichtung zum Mischen und Austragen von Knochenzement vorgeschlagen, die in dem Zementiersystem Palacos® PRO verwirklicht wurde, bei der ein gasdurchlässiger Sterilisationskolben und ein Dichtungskolben axial beweglich an einer Mischstange angeordnet sind. In der Sterilisationsstellung verschließt der Sterilisationskolben die Kartusche so, dass kein Zementpulver austreten kann, aber durch eine gasdurchlässige Austauschfläche des Sterilisationskolbens Ethylenoxid in die Kartusche zur Sterilisation des Zementpulvers und der Innenseite der Kartusche einströmen kann und nach erfolgter Sterilisation wieder aus dem Innenraum der Kartusche heraus diffundieren kann. Der Sterilisationskolben enthält eine umlaufende Nut, in die ein umlaufender Steg der Kartusche greift, der an der Innenseite der Kartusche angeordnet ist. Dadurch ist der Sterilisationskolben mit der Kartusche reversibel verbunden und der Sterilisationskolben ist fixiert.

Vor dem Mischen des Knochenzements wird der Dichtungskolben auf beziehungsweise in den Sterilisationskolben gesteckt. Dadurch bildet sich ein aus zwei Teilen bestehender Kolben. Wie auch bei einem erfindungsgemäßen Verfahren wird Vakuum an den Dichtungskolben angelegt, und der Monomertransfer aus dem Monomerreservoir in das Innere der Kartusche zum dort befindlichen Zementpulver wird durch Betätigung eines Öffnungsmechanismus gestartet. Anschließend wird mit Hilfe der Mischstange und einem daran befindlichen Mischelement das Zementpulver mit der Monomerflüssigkeit vermischt. Danach wird kein Vakuum weiter gezogen. Die Mischstange wird in Richtung des zweiteiligen Kolbens gezogen bis das Mischelement an der Unterseite des zweiteiligen Kolbens anliegt. Dann wird der Mischstab unmittelbar oberhalb der Oberseite des zweiteiligen Kolbens an einer Sollbruchstelle abgebrochen. Die Kartusche mit dem gebildeten Zementteig wird dem Fußteil gelöst. Dann wir ein Austragsrohr an den Kartuschenkopf angebracht und die Kartusche mit einer Austragsvorrichtung verbunden.

Bei der ersten Bewegung des Stößels der Austragsvorrichtung auf den zweiteiligen Kolben wird der Nut-Steg-Verbindung des Sterilisationskolbens mit der Kartusche durch Überfahren des Stegs der Kartusche gelöst und der zweiteilige Kolben ist dann axial in Richtung Kartuschenkopf verschiebbar. Die Kartusche wird annähernd senkrecht gehalten und die Austragsvorrichtung presst den zweiteiligen Kolben axial in Richtung Kartuschenkopf, wobei die in der Kartusche enthaltene Luft über das Austragsrohr entwicht bis der Zementteig das Austragsrohr erreicht. Danach wird der Zementteig appliziert.

Die WO 2009/105905 A1 offenbart eine Spritze mit einer Mischvorrichtung gemäß dem Oberbegriff des Anspruchs 1 umfassend einen Mischstab, wobei der Mischstab durch einen Kolben geführt ist. Die Bewegung des Kolbens gegen den Mischstab lässt sich durch einen schwenkbaren Hebel am Mischstab einschränken. Ferner offenbart die WO 2009/105905 A1 ein Verfahren zum Spritzen mit einer Vorrichtung nach dem Oberbegriff des Anspruchs 1 mit den folgenden Verfahrensschritten:
A) Bereitstellen einer mit einem Pulver bereichsweise gefüllten Kartusche;
D) Einfüllen eines Fluids in die Kartusche;
E) Mischen des Pulvers mit dem Fluid mit einem Mischelement;
F) Verriegeln des Mischstabs gegen den Kolben mit dem schwenkbaren Hebel;
H) Austragen der Mischung durch Vortreiben des Kolbens.

In der DE 43 02 230 A1 wird eine Mischvorrichtung für Knochenzement offenbart, bei der ein Vakuumanschluss am Kartuschenkopf vorhanden ist und die einen reversibel fixierten Kolben am Kartuschenende besitzt. Nach erfolgter Vermischung der Pulverkomponente mit der Monomerflüssigkeit wird der durch einen Drehverschluss fixierte Kolben durch eine relative Drehbewegung der Kartusche gegenüber dem Kolben gelöst. Der Kolben besitzt dazu Aussparungen an seiner Außenseite mit denen er in Sicken eines speziellen Blisters eingreifen kann und dadurch bei einer Drehung der Kartusche um ihre Längsachse fixiert ist. Der so gelöste Kolben bewegt sich in Folge der Einwirkung des anliegenden Vakuums in Richtung Kartuschenkopf und sammelt bei dieser Bewegung den Zementteig und transportiert ihn zum Kartuschenkopf.

Die in der DE 10 2009 031 178 B3 offenbarte Mischvorrichtung ermöglicht nicht das Sammeln des gemischten Zementteigs unter Vakuum durch Bewegung des Kolbens in Richtung Kartuschenkopf. Ein Sammeln unter Vakuum kann nicht erfolgen, weil einerseits die Lösung der Fixierung des Sterilisationskolbens an der Kartusche nur durch Einwirkung des Stößels des Zementapplikators erfolgen kann, eine manuelle Entriegelung nicht möglich ist, und andererseits weil bei Vakuumeinwirkung der abgebrochene Mischstab in das Innere der Kartusche gesaugt wird.

Die Aufgabe der Erfindung besteht also darin, die Nachteile des Stands der Technik ausgehend von der DE 10 2009 031 178 B3 zu überwinden, das heißt, eine Mischvorrichtung zu entwickeln, welche die dargelegten Probleme der in DE 10 2009 031 178 B3 offenbarten Mischvorrichtung und weitere nicht genannte Nachteile, die sich aber ohne weiteres aus dem Stand der Technik ergeben, überwindet. Insbesondere soll eine Mischvorrichtung bereitgestellt werden, die eine geschlossene Mischvorrichtung ist, so dass vor und während des Anmischens des Zementteigs weder das Monomer noch das Zementpulver direkten Kontakt mit der Umgebung haben und dadurch keine Kontamination der Zementkomponenten erfolgen kann. Die Mischvorrichtung soll einen Kolben besitzen, der nach erfolgtem Mischen der Zementkomponenten in der Kartusche unter Einwirkung von Vakuum in Richtung Kartuschenkopf bewegt werden kann, wobei der gebildete Zementteig gesammelt und in Richtung Kartuschenkopf bewegt wird. Auch soll während des Sammelns des Zementteigs unter Vakuum der Mischstab nicht durch Vakuumeinwirkung in das Innere der Kartusche gesaugt werden können.

Die Aufgaben der Erfindung werden gelöst durch eine Vorrichtung gemäß Anspruch 1 zum Mischen und Austragen einer pastösen Masse, insbesondere eines Knochenzements, aufweisend eine an zwei Enden offene oder zu öffnende Kartusche, in der ein Mischelement und ein Förderkolben angeordnet sind, wobei der Förderkolben in einer Ausgangsstellung im Bereich eines ersten Kartuschenendes angeordnet ist, das Mischelement an einem Mischstab angeordnet ist, der Mischstab sich durch den Förderkolben in das Kartuscheninnere erstreckt, der Mischstab und der Förderkolben mit der Kartusche eine dichte Verbindung bilden, die das Kartuscheninnere der Vorrichtung nach außen abdichtet, wobei der Förderkolben auf dem Mischstab und in der Kartusche axial bewegbar angeordnet ist und wobei wenigstens eine Klemmbacke am Förderkolben derart angeordnet ist, dass die wenigstens eine Klemmbacke mit einem Verriegelungselement derart an den Mischstab anpressbar ist, dass der Mischstab nicht mehr gegen den Förderkolben verschiebbar ist, wobei das Verriegelungselement ein Teil des Förderkolbens ist, das auf dem Mischstab axial gegen den restlichen Förderkolben verschiebbar ist und das bei einem Zusammenschieben oder Zusammenstecken des Verriegelungselements mit dem restlichen Förderkolben die wenigstens eine Klemmbacke an den Mischstab presst, so dass der Mischstab nicht mehr gegen den Förderkolben verschiebbar ist.

Die Klemmbacke bildet erfindungsgemäß bevorzugt eine flächige Verbindung mit dem Mischstab. Die Klemmfläche kann dabei gezahnt sein. Alternativ kann die Klemmbacke auch als Dorn oder mit einer Kante ausgebildet sein, die beim anpressen in die Oberfläche des Mischstabs eingedrückt werden. Grundsätzlich ist es auch vorstellbar, dass die Klemmbacke als Band aufgebaut ist, das durch ein Zuschnüren den Mischstab an den Förderkolben klemmt beziehungsweise schnürt.

Dass der Mischstab nicht mehr verschiebbar ist, bedeutet nicht, dass er nicht durch eine nicht vorgesehene Krafteinwirkung verschiebbar wäre, sondern dass er bei einer normalen Anwendung der Vorrichtung nicht mehr gegen den Förderkolben verschiebbar ist. Insbesondere soll der Mischstab nicht mehr durch Vakuumeinwirkung im Kartuscheninneren gegen den Förderkolben verschiebbar sein.

Als axiale Richtung wird erfindungsgemäß die Richtung verstanden, in die der Mischstab verschiebbar ist und die eine Symmetrieachse der Innenwand der Kartusche oder der Kartusche als Ganzes (zumindest im Wesentlichen) bildet. Erfindungsgemäß ist bevorzugt, dass die Kartuscheninnenwände zylindrisch sind, vorzugsweise die Kartusche zylindrisch ist. Dann ist die Zylinderachse der Kartusche beziehungsweise deren Kartuischeninnenwände bevorzugt die Achse auf die sich die axiale Richtung bezieht.

Es kann vorgesehen sein, dass die Klemmbacke oder zumindest eine der Klemmbacken einen Rasthaken aufweisen, der in ein Gegenrastmittel am Verriegelungselement einrastet, wenn das Verriegelungselement auf die Klemmbacken geschoben oder gesteckt wird beziehungsweise ist.

Es kann erfindungsgemäß bevorzugt vorgesehen sein, dass das Mischelement tiefer im Kartuscheninneren angeordnet ist, als der Förderkolben in seiner Ausgangsstellung. Die Ausgangsstellung des Förderkolbens ist diejenige Stellung, bei der die pastöse Mischung noch nicht aus dem Kartuscheninneren herausgepresst ist. Ebenso kann erfindungsgemäß vorgesehen sein, dass sich das Mischelement vor der Seite des Förderkolbens angeordnet ist, in deren Richtung der Förderkolben bewegt wird, um die pastöse Masse aus dem Kartuscheninneren herauszupressen. Diese Richtung wird als die nach innen weisende Richtung (zum inneren der Kartusche hin) bezeichnet. Die entgegengesetzte Richtung wird als die nach außen weisende Richtung bezogen auf den Förderkolben bezeichnet.

Gemäß einer Weiterbildung der Erfindung kann auch vorgesehen sein, dass das Verriegelungselement ein separates Teil des Förderkolbens ist, das auf dem Mischstab axial gegen den restlichen Förderkolben verschiebbar ist und das bei einem Zusammenschieben oder Zusammenstecken des Verriegelungselements mit dem restlichen Förderkolben die wenigstens eine Klemmbacke an den Mischstab presst, so dass der Mischstab nicht mehr gegen den Förderkolben verschiebbar ist.

Dieser Aufbau ist kostengünstig und einfach zu realisieren. Zudem ist mit diesem Aufbau eine einfache Bedienung der Vorrichtung möglich.

Mit einer besonders bevorzugten Weiterentwicklung der Erfindung wird vorgeschlagen, dass zwischen dem Verriegelungselement und dem restlichen Förderkolben ein manuell entfernbarer Abstandhalter als Sicherungselement angeordnet ist, der ein unbeabsichtigtes Zusammenschieben oder Zusammenstecken des Verriegelungselements und des restlichen Förderkolbens verhindert.

Der Abstandhalter verhindert auf kostengünstige und leicht zu realisierende Weise eine ungewollte Verriegelung des Mischstabs mit dem Förderkolben und beugt somit einer Fehlbedienung der Vorrichtung im oft hektischen Anwendungsfall zum Beispiel bei einer Operation vor.

Ferner ist bevorzugt vorgesehen, dass das Verriegelungselement an dem nach außen weisenden Ende des Förderkolbens angeordnet ist.

Hierdurch werden die Bedienbarkeit und der Aufbau der Vorrichtung vereinfacht.

Gemäß einer Weiterbildung der Erfindung kann vorgesehen sein, dass das Verriegelungselement durch Zusammenschieben oder Aufstecken auf den restlichen Förderkolben, formschlüssig oder kraftschlüssig derart mit dem Förderkolben, insbesondere mit dem Dichtungskolben des Förderkolbens verbindbar ist, dass die mindestens eine Klemmbacke an den Mischstab gepresst wird. Ferner kann vorgesehen sein, dass das Verriegelungselement mit mindestens einem Rastelement unlösbar mit dem Dichtungskolben verbindbar ist.

Es kann erfindungsgemäß auch vorgesehen sein, dass der Förderkolben an dem ins Kartuscheninnere weisenden Ende einen Sterilisationskolben aufweist, neben dem das Mischelement in der Kartusche angeordnet ist, wobei der Sterilisationskolben mindestens eine gasdurchlässige Austauschfläche aufweist und vorzugsweise mit wenigstens einem lösbaren Rastelement mit der Kartusche verbunden oder verbindbar ist.

Durch den Sterilisationskolben wird eine Desinfizierung des Kartuscheninneren mit einem sterilisierenden Gas wie Ethylenoxid ermöglicht. Dies ist vor allem für eine Anwendung im klinischen Bereich von zentraler Bedeutung.

Des Weiteren kann vorgesehen sein, dass der Förderkolben einen Dichtungskolben aufweist, insbesondere als separates Teil des restlichen Förderkolbens, wobei der Dichtungskolben auf dem Mischstab axial verschiebbar angeordnet ist und an dem Dichtungskolben die wenigstens eine Klemmbacke angeordnet ist.

Der Dichtungskolben hat die zentrale Aufgabe die Kartusche in einer Richtung abzudichten und sorgt auch dafür, dass bei dem Auspressen des Kartuscheninhalts (der pastösen Masse) dieser nicht an dem Dichtungskolben vorbei ungewollt entgegen der Pressrichtung entweichen kann. Der vorgeschlagene separate beziehungsweise modulare Aufbau des Förderkolbens bewirkt eine kostengünstige und einfache Möglichkeit den erfindungsgemäßen Aufbau zu realisieren.

Bei erfindungsgemäßen Vorrichtungen mit Sterilisationskolben und Dichtungskolben kann vorgesehen sein, dass der Sterilisationskolben mit dem Dichtungskolben und dem Verriegelungselement nach Zusammenschieben oder Zusammenstecken einen zumindest zweiteiligen Förderkolben bildet, vorzugsweise zusammen mit dem Verriegelungselement einen zumindest dreiteiligen Förderkolben bildet.

Dieser modulare Aufbau des Förderkolbens ermöglicht die Verwendung einfacher und damit kostengünstig zu fertigender Einzelteile. Zudem ist durch den einfachen Aufbau die Gefahr einer Fehlfunktion reduziert.

Dabei kann auch vorgesehen sein, dass der Dichtungskolben auf den Sterilisationskolben aufsteckbar ist und dass das Verriegelungselement auf einen aus dem Dichtungskolben und dem Sterilisationskolben zusammengesetzten Förderkolbenteil unter Bildung eines dreiteiligen Förderkolbens aufsteckbar oder aufschiebbar ist.

Dieser Aufbau ist besonders leicht und einfach anzuwenden.

Mit einer Weiterbildung der Erfindung wird auch vorgeschlagen. dass der restliche Förderkolben, insbesondere der Dichtungskolben mindestens eine Ausnehmung an der Außenseite zur Aufnahme eines Griffstücks des mindestens einen Rastmittels des Sterilisationskolbens aufweist.

Die Ausnehmung bewirkt eine manuelle Bedienbarkeit der Vorrichtung, beispielsweise mit zwei Fingern, bei gleichzeitig kompakten und platzsparenden Aufbau der Vorrichtung.

Es kann erfindungsgemäß bevorzugt vorgesehen sein, dass der Sterilisationskolben und/oder der Dichtungskolben ein zum restlichen Förderkolben separates Teil ist oder sind.

Die vereinzelten Teile sind leichter und kostengünstiger zu fertigen, als wenn diese miteinander verbunden wären. Die Teile des mehrteiligen Förderkolbens können aber auch über flexible Verbindungen fest miteinander verbunden sein, ohne dass dies die Funktionalität für die vorliegende Erfindung maßgeblich einschränkt.

Es kann erfindungsgemäß auch vorgesehen sein, dass das dreiteilige Förderkolbensystem den Mischstab umschließt.

Gemäß einer besonders bevorzugten Weiterbildung der Erfindung kann vorgesehen sein, dass der Förderkolben, insbesondere das dreiteilige Förderkolbensystem axial in die Kartusche, insbesondere in einen Mischzylinder der Kartusche, nach einer Entriegelung durch einen Druck verschiebbar ist, um einen aus Knochenzementpulver und einem Monomer gemischten Knochenzementteig aus dem Kartuscheninneren in Richtung eines Kartuschenkopfs zu bewegen, der an einem, dem ersten Ende der Kartusche gegenüber liegenden zweiten Ende der Kartusche angeordnet ist und der eine zweite Öffnung beinhaltet.

Hierdurch ist der dreiteilige Förderkolben als Förderkolben für pastöse Massen einsetzbar.

Dabei kann erfindungsgemäß vorgesehen sein, dass die Austragsöffnung im Kartuschenkopf ein Anschlussmittel, insbesondere ein Anschlussgewinde, aufweist.

Auch kann vorgesehen sein, dass der Mischstab eine Sollbruchstelle aufweist, wobei bevorzugt die Solbruchstelle des Mischstabs derart angeordnet ist, dass die Sollbruchstelle durch einen Abstandhalter verdeckt ist, wenn das Mischelement mit dem Mischstab bis an das ins Kartuscheninnere weisende Ende des Förderkolbens gezogen ist.

Mit der Sollbruchstelle werden ein reproduzierbares Abbrechen des Mischstabs und damit eine verlässliche Anwendung der Vorrichtung ermöglicht. Wenn die Sollbruchstelle durch den Abstandhalter verdeckt ist, wird dadurch ein ungewolltes Abbrechen des Mischstabs verhindert, da der Abstandhalter die Sollbruchstelle stützt und eventuell auftretende Drehmomente weiter Außen am Mischstab angreifen, an denen der Mischstab aus dem Förderkolben geführt ist. Erst nach dem Entfernen des Abstandhalters und gegebenenfalls nach eindrücken beziehungsweise einstecken des Verriegelungselements wird die Sollbruchstelle freigelegt und der Mischstab an dieser Stelle leicht abbrechbar.

Es kann erfindungsgemäß auch vorgesehen sein, dass der Förderkolben, insbesondere der Dichtungskolben und/oder der Sterilisationskolben wenigstens einen Vakuumanschluss aufweist, der eine Verbindung durch den Förderkolben von außen ins Kartuscheninnere bereitstellt.

Mit Hilfe des Vakuumanschluss kann ein sterilisierendes Gas aus der Kartusche entfernt werden, eine fluide Komponente des pastösen Gemischs in die Kartusche eingesaugt werden und auch der Förderkolben ins Kartuscheninnere vorgetrieben werden.

Mit einer bevorzugten Ausführungsform der erfindungsgemäßen Vorrichtung mit Vakuumanschluss wird vorgeschlagen, dass ein Rückschlagventil in dem Vakuumanschluss angeordnet ist.

Mit einer Weiterbildung der Erfindung wird vorgeschlagen, dass die zumindest eine Klemmbacke kippbar am Förderkolben, insbesondere am Dichtungskolben angeordnet ist und mit ihrem oder ihren dem Boden des Förderkolbens abgewandten Ende oder Enden durch Kippen in Richtung der Längsachse des Mischstabs an den Mischstab pressbar ist oder sind.

Es kann ferner vorgesehen sein, dass das Verriegelungselement mindestens eine kippbare Klemmbacke aufweist, die mit ihrem dem Drehpunkt der Klemmbacke abgewandten Ende durch Kippen in Richtung der Längsachse des Mischstabs an den Mischstab pressbar ist.

Derart aufgebaute Klemmbacken sind kostengünstig zu realisieren und zudem robust und erlauben so eine zuverlässige Anwendung der Vorrichtung.

Ferner wird mit der Erfindung vorgeschlagen, dass das Verriegelungselement und/oder der Dichtungskolben mindestens ein elastisch verformbares Element aufweist, das nach Zusammenschieben oder Aufstecken des Verriegelungselements auf den restlichen Förderkolben, insbesondere den Dichtungskolben die mindestens eine Klemmbacke an den Mischstab presst.

Durch das elastische Element wird eine sichere Verbindung der Klemmbacke mit dem Mischstab bereitgestellt.

Die Aufgaben der Erfindung werden auch gelöst durch ein Knochenzementsystem aufweisend eine solche Vorrichtung, ein Vorratselement für einen Binder, insbesondere einem Monomer, und ein Basiselement, wobei das Basiselement die Vorrichtung und das Vorratselement lagert und verbindet.

Das Knochenzementsystem hat alle Bauteile zum Bereitstellen eines Knochenzements.

Dabei kann vorgesehen sein, dass das Basiselement ein Koppelmittel für eine kraft- und/oder formschlüssige Verbindung mit der Vorrichtung aufweist, insbesondere eine kraft- und/oder formschlüssige Verbindung mit der Austragsöffnung der Vorrichtung.

Die Vorrichtung kann so einfach in das Knochenzementsystem eingesetzt werden.

Ferner kann vorgesehen sein, dass das Vorratselement ein Ventilmittel, insbesondere ein manuell bedienbares Ventilmittel aufweist, um einen Ausfluss eines Monomers aus dem Vorratselement zu steuern und/oder auszulösen.

Hierdurch kann der Ausfluss des Knochenzements aus dem Knochenzementsystem gesteuert werden.

Ferner kann bei erfindungsgemäßen Knochenzementsystemen vorgesehen sein, dass das Basiselement eine Leitung aufweist, wobei die Leitung das Vorratselement mit dem Kartuscheninneren verbindet, so dass ein Monomer aus dem Vorratselement durch die Leitung in die Kartusche strömen kann.

Das Knochenzementsystem ist dann zum Herstellen der Knochenzementmischung geeignet.

Bei erfindungsgemäßen Knochenzementsystemen kann auch vorgesehen sein, dass das Vorratselement einen Vorratsbehälter für den Binder lagert, wobei bevorzugt vorgesehen sein kann, dass der Vorratsbehälter ein Glasbehälter ist.

Der Glasbehälter ist chemisch gegen den Binder resistent und kann leicht durch Brechen geöffnet werden.

Die der Erfindung zugrundeliegenden Aufgaben werden auch gelöst durch ein Verfahren gemäß Anspruch 20 zum Herstellen eines Knochenzements mit einer solchen Vorrichtung oder einem solchen Knochenzementsystem, mit den folgenden chronologischen Verfahrensschritten:
A) Bereitstellen einer mit einem Pulver zumindest bereichsweise gefüllten Kartusche;
D) Füllen der Kartusche mit einem Fluid, bevorzugt mit einem Binder, besonders bevorzugt mit einem Monomer;
E) Mischen des Pulvers mit dem Mischelement;
F) Verriegeln des Mischstabs im Förderkolben mit der zumindest einen Klemmbacke; und
H) Austragen des Zementgemischs durch Vortreiben des Förderkolbens.

Ferner kann ein zusätzlich Verfahrensschritt B) vorgesehen sein:
B) Einfüllen eines sterilisierenden Gases durch eine gasdurchlässige Austauschfläche und Sterilisieren des Kartuscheninnenraums und des Pulvers nach Schritt A) und vor Schritt D).

Zudem kann vorgesehen sein, dass bei Schritt A) die Kartusche durch einen Sterilisationskolben verschlossen ist.

Durch diese Maßnahmen kann ein steriler Zement beziehungsweise eine sterile pastöse Masse bereitgestellt werden.

Erfindungsgemäße Verfahren können auch weitergebildet werden durch einen Schritt C) Einstecken eines Dichtungskolbens in den Sterilisationskolben nach Schritt B) und vor Schritt D).

Ferner kann ein Schritt G) mit Abbrechen des mit der wenigstens einen Klemmbacke verriegelten Mischstabs nach Schritt F) und vor Schritt H), wobei vorzugsweise der Mischstab an einer Sollbruchstelle abgebrochen wird, vorgesehen sein.

Gemäß einer Weiterbildung des erfindungsgemäßen Verfahrens kann vorgesehen sein, dass bei Schritt F) der Mischstab durch ein Aufschieben oder Aufstecken des Verriegelungselements auf den restlichen Förderkolben, insbesondere auf den Dichtungskolben verriegelt wird, wobei das Verriegelungselement durch das Aufschieben oder Aufstecken die wenigstens eine Klemmbacke auf den Mischstab presst.

Dabei kann vorgesehen sein, dass bei Schritt F) vor dem Aufschieben oder Aufstecken des Verriegelungselements ein Abstandhalter als Sicherungselement entfernt wird.

Schließlich kann erfindungsgemäß auch vorgesehen sein, dass bei Schritt H) vor dem Austragen des Zementgemischs der Förderkolben, insbesondere der mehrteilige Förderkolben durch Lösen wenigstens eines Rastelements von der Kartusche gelöst wird.

Der Erfindung liegt die überraschende Erkenntnis zugrunde, dass durch die Verwendung einer Klemmbacke oder einer äquivalenten Klemmvorrichtung, die erfindungsgemäß ebenfalls als Klemmbacke verstanden werden kann, gelingt, eine Vorrichtung bereitzustellen, bei der eine Gemisch mit einem Mischelement in einer Kartusche mit Hilfe eines Mischstabs gemischt werden kann, wobei der Mischstab, wenn er für eine weitere Anwendung der Vorrichtung nicht mehr gebraucht wird und eher hinderlich wäre, einfach und reproduzierbar abgebrochen werden kann, ohne dass die Funktionalität der Vorrichtung beeinträchtigt wäre.

Insbesondere durch einen mehrteiligen Aufbau des Förderkolbens ist eine kostengünstige und leicht zu bedienende Vorrichtung realisiserbar.

Die Mischvorrichtung hat einen manuell entriegelbaren Förderkolben, der nach erfolgtem Mischen der Zementkomponenten in der Kartusche unter Einwirkung von Vakuum in Richtung Kartuschenkopf bewegt werden kann, wobei der gebildete Zementteig (die pastöse Masse) gesammelt und in Richtung Kartuschenkopf bewegt wird. Die Mischvorrichtung ist so gestaltet, dass nach erfolgtem Mischen, der abzubrechende Mischstab am Förderkolben festgelegt werden kann, so dass dieser während des Sammelns des Zementteigs unter Vakuum nicht durch Vakuumeinwirkung in das Innere der Kartusche gesaugt werden kann.

Die Aufgaben der Erfindung werden gelöst durch eine Vorrichtung zum Mischen und Austragen von Knochenzement, mit einer zylinderförmigen Kartusche, in welcher ein Mischelement angeordnet ist, wobei das Mischelement mittels einer an einem ersten Kartuschenende gedichtet herausgeführten Mischstab axial bewegbar ist, und mit einem im Bereich des ersten Kartuschenendes angeordneten und auf dem Mischstab axial bewegbaren Dichtungskolben, der die Kartusche gasdicht abschließt, wobei
a) im Bereich des ersten Zylinderendes zwischen dem Mischelement und dem Dichtungskolben ein Sterilisationskolben angeordnet ist, der mindestens eine gasdurchlässige Austauschfläche besitzt und der mit wenigstens einem von außen manuell lösbaren Rastelement mit der Kartusche reversibel verbunden ist,
b) auf dem Mischstab der getrennt vom Sterilisationskolben axial verschiebbarer Dichtungskolben angeordnet ist,
c) wenigstens eine Klemmbacke am Dichtungskolben und/oder an einem separaten Verriegelungselement so angeordnet ist, dass diese an den Mischstab angepresst werden kann,
d) ein Verriegelungselement angeordnet ist, das auf dem Mischstab axial verschiebbar ist, das beim Zusammenschieben mit dem Dichtungskolben die wenigstens eine Klemmbacke so an den Mischstab presst, dass dieser nicht mehr durch Vakuumeinwirkung verschiebbar ist, und wobei der Sterilisationskolben mit dem Dichtungskolben und dem Verriegelungselement nach Zusammenschieben oder Zusammenstecken einen dreiteiligen Förderkolben bildet.

Die Funktionsweise der erfindungsgemäßen Vorrichtung wird nachfolgend genauer erklärt. Der Sterilisationskolben befindet sich in der Kartusche unmittelbar neben dem Kartuschenboden und ist mit dem Rastmittel an der Innenseite der Kartusche fixiert. In einer Aussparung des Sterilisationskolbens befindet sich der Mischstab der axial durch die Aussparung bewegt werden kann. An dem im Inneren der Kartusche befindlichen Ende des Mischstabs ist ein Mischelement angeordnet. Vorzugsweise können Mischflügel als Mischelemente verwendet werden. An dem äußeren Ende des Mischstabs ist ein Handgriff angeordnet. Der Sterilisationskolben enthält eine gasdurchlässige Austauschfläche. Vom Sterilisationskolben getrennt befinden sich ein Dichtungskolben und ein Verriegelungselement, die jeweils um den Mischstab axial beweglich angeordnet sind.

In der Sterilisationsstellung ist der Sterilisationskolben getrennt von dem Dichtungskolben und dem Verriegelungselement, wobei der Sterilisationskolben als einziger schon in die Kartusche eingesetzt und in die Kartusche eingerastet ist. Das gasförmige Sterilisationsmittel (beispielsweise und bevorzugt Ethylenoxid) kann durch die gasdurchlässige Austauschfläche in das Innere der Kartusche eindringen und das darin befindliche Zementpulver und die Innenseite der Kartusche sterilisieren. Anschließend diffundiert das Sterilisationsmittel aus der Kartusche durch die gasdurchlässige Austauschfläche. Damit dieser Entgasungsprozess in einer wirtschaftlich vertretbaren Zeit abläuft, muss die Austauschfläche ausreichend groß sein. Bevorzugt nimmt die Austauschfläche zumindest 25% der Querschnittsfläche der Kartusche ein, besonders bevorzugt zumindest 40%, ganz besonders bevorzugt zumindest 50%.

Beim Herstellen eines medizinischen Zements, insbesondere eines Knochenzements, kann die Kartusche als Zementkartusche ausgebildet sein, das heißt, als Kartusche ausgebildet sein, die eine ausreichend Stabilität aufweist, dass ein pastöser Zement in ihr gemischt und aus der Zementkartusche ausgetragen werden kann. Zudem muss die Kartusche ausreichend chemisch stabil sein, so dass keine störenden Substanzen aus den Wänden der Zementkartusche gelöst werden. Dies ist insbesondere bei Zementkartuschen für medizinisch anzuwendende Zemente wie Knochenzemente sehr wichtig.

Zum Anmischen des Zements wird der Dichtungskolben auf beziehungsweise in den Sterilisationskolben geschoben oder gesteckt, so dass die Kartusche gasdicht von der umgebenden Atmosphäre abgeschlossen ist und dass die Aussparungen des Dichtungskolbens die Griffstücke des Sterilisationskolbens aufnehmen. Der Dichtungskolben enthält einen Vakuumanschluss der mit dem Innenraum der Kartusche gasdurchlässig verbunden ist. Es wird dann mit einem Vakuumschlauch über den Vakuumanschluss der Innenraum der Kartusche evakuiert. Danach wird unter Vakuumeinwirkung das Monomer aus einem Vorratsbehälter in die Kartusche überführt. Beispielsweise wird das Monomer durch die Vakuumeinwirkung eingesaugt. Dann werden unter Vakuum mit dem am Mischstab befindlichen Mischelement die Zementkomponenten solange gemischt bis ein homogener Zementteig entstanden ist. Anschließend wird der Mischstab nach oben in Richtung des Sterilisationskolbens gezogen bis das Mischelement an der Unterseite des Sterilisationskolbens anliegt. Das Vakuum wird kurzzeitig abgestellt. Dann wird das Verriegelungselement manuell auf den Dichtungskolben geschoben beziehungsweise gepresst. Durch das Verriegelungselement wird die Klemmbacke oder werden die Klemmbacken an den Mischstab gepresst und der Mischstab wird durch die mindestens eine Klemmbacke festgelegt. Dann wird der obere Teil der Mischstange über eine Sollbruchstelle abgebrochen. Dann wird wieder Vakuum angelegt und das mindestens eine Griffstück in Richtung der Kartuschenlängsachse bewegt. Dadurch wird das Rastelement von der Innenseite der Kartusche gelöst und der gesamte Kolben, beinhaltend den Sterilisationskolben und den Dichtungskolben, bewegt sich infolge der Einwirkung des Vakuums in Richtung Kartuschenkopf, wobei der zuvor gemischte Zementteig gesammelt und in Richtung Kartuschenkopf mitgenommen wird. Dann wir der Vakuumschlauch abgezogen und die Kartusche ist dann zur Zementapplikation bereit.

Die gesamte Vorrichtung kann bevorzugt aus Kunststoffen gebildet sein, wobei thermoplastische Kunststoffe besonders kosteneffizient einsetzbar und daher erfindungsgemäß besonders bevorzugt sind. Mit diesen Kunststoffen kann die erfindungsgemäße Vorrichtung durch übliches Kunststoffspritzgießen gefertigt werden.

Im Folgenden werden Ausführungsbeispiele der Erfindung anhand von neun schematisch dargestellten Figuren erläutert, ohne jedoch dabei die Erfindung zu beschränken. Dabei zeigt:
- Figur 1:: eine schematische perspektivische Ansicht eines Förderkolbens mit einem Mischelement und einem Mischstab für eine erfindungsgemäße Vorrichtung;
- Figur 2:: eine schematische Querschnittansicht durch eine erfindungsgemäße Vorrichtung;
- Figur 3:: eine schematische Querschnittansicht durch eine erfindungsgemäße Vorrichtung senkrecht zu Figur 2;
- Figur 4:: eine schematische perspektivische Ansicht eines Sterilisationskolbens für eine erfindungsgemäße Vorrichtung;
- Figur 5:: eine schematische perspektivische Ansicht eines Dichtungskolbens für eine erfindungsgemäße Vorrichtung;
- Figur 6:: eine schematische perspektivische Ansicht eines Verriegelungselements für eine erfindungsgemäße Vorrichtung;
- Figur 7:: eine schematische Seitenansicht eines Förderkolbens mit Sicherungselement und nicht arretiertem Mischstab;
- Figur 8:: eine schematische Seitenansicht eines Förderkolbens ohne Sicherungselement und arretiertem Mischstab; und
- Figur 9:: eine schematische Querschnittansicht eines erfindungsgemäßen Knochenzementsystems.

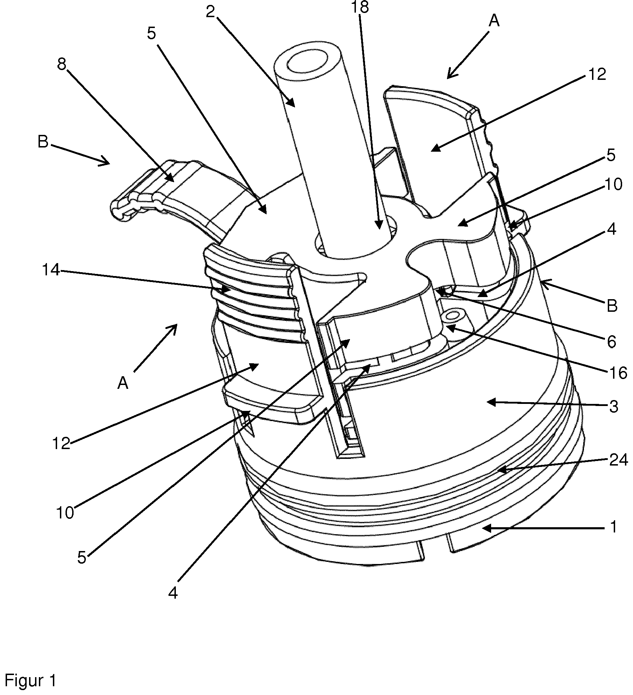

Figur 1 zeigt eine schematische perspektivische Ansicht eines Förderkolbens mit einem Mischelement 1 und einem Mischstab 2 für eine erfindungsgemäße Vorrichtung. Der Förderkolben hat zylindrische Außenabmessungen ist zum Einsetzen in eine Kartusche (nicht gezeigt) mit zylindrischer Innenwand vorgesehen. Der Förderkolben ist aus drei separaten Teilen aufgebaut. Das Mischelement 1 ist im eingebauten Zustand im Inneren der Kartusche angeordnet und dient dazu, den Kartuscheninhalt zu durchmischen. Dazu kann das Mischelement 1 über den Mischstab 2 in Längsrichtung der Kartusche bewegt werden und um den Mischstab 2 als Drehachse gedreht werden.

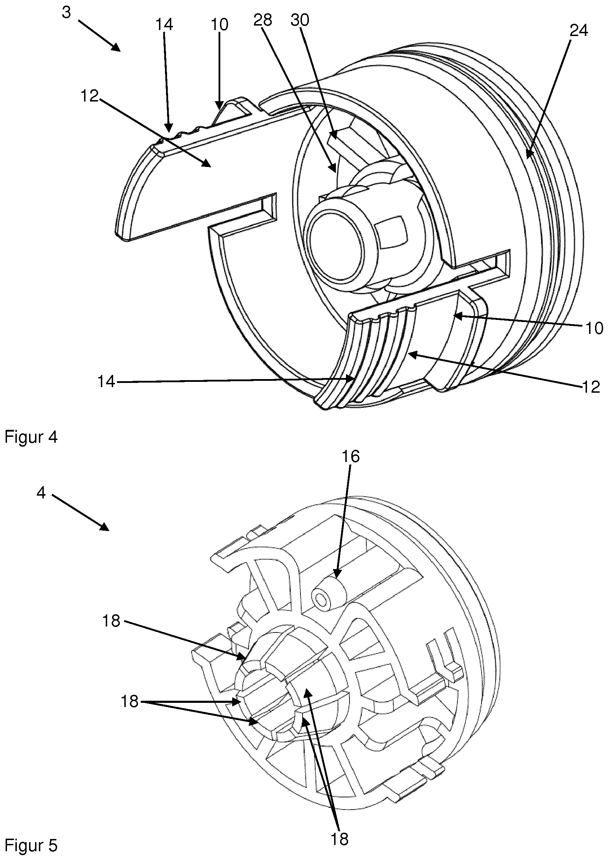

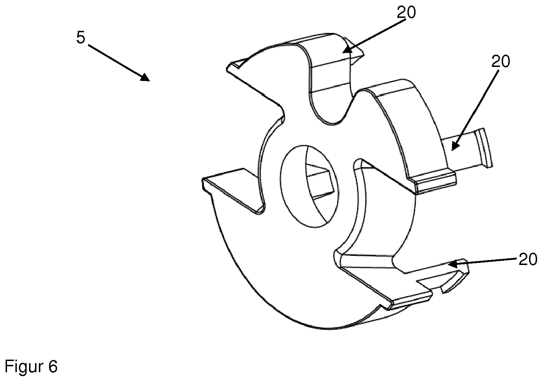

Der Förderkolben weist einen äußeren Sterilisationskolben 3 auf, der den zum Mischelement 1 angrenzenden Teil des Förderkolbens bildet. Der Sterilisationskolben 3 ist oben auf dem Mischelement 1 angeordnet. Der Sterilisationskolben 3 ist einzeln in Figur 4 dargestellt. In und auf dem Sterilisationskolben 3 ist ein Dichtungskolben 4 als zweiter Teil des Förderkolbens angeordnet. Der Dichtungskolben 4 ist einzeln in Figur 5 dargestellt. Oberhalb des Dichtungskolbens 4 ist ein Verriegelungselement 5 als dritter Teil des Förderkolbens angeordnet. Das Verriegelungselement 5 ist einzeln in Figur 6 dargestellt.

Wann immer im Folgenden Details zum Sterilisationskolben 3, dem Dichtungskolben 4 und dem Verriegelungselement 5 diskutiert werden, sei dabei auch auf die Figuren 4, 5 und 6 verwiesen.

Zwischen dem Dichtungskolben 4 und dem Verriegelungselement 5 ist ein Abstandhalter 6 als Sicherungselement 6 angeordnet, mit dem verhindert wird, dass das Verriegelungselement 5 auf den Dichtungskolben 4 gesteckt wird und dadurch den Mischstab 2 versehentlich verriegelt. Der Abstandhalter 6 weist einen Griff 8 auf, mit dem er zwischen dem Dichtungskolben 4 und dem Verriegelungselement 5 herausziehbar ist, auch wenn der Förderkolben in der Kartusche angeordnet ist.

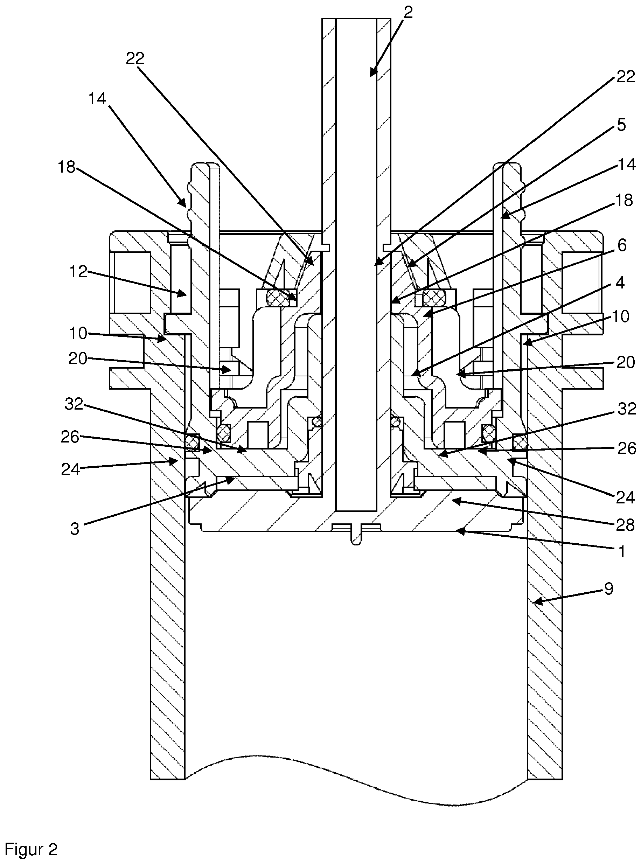

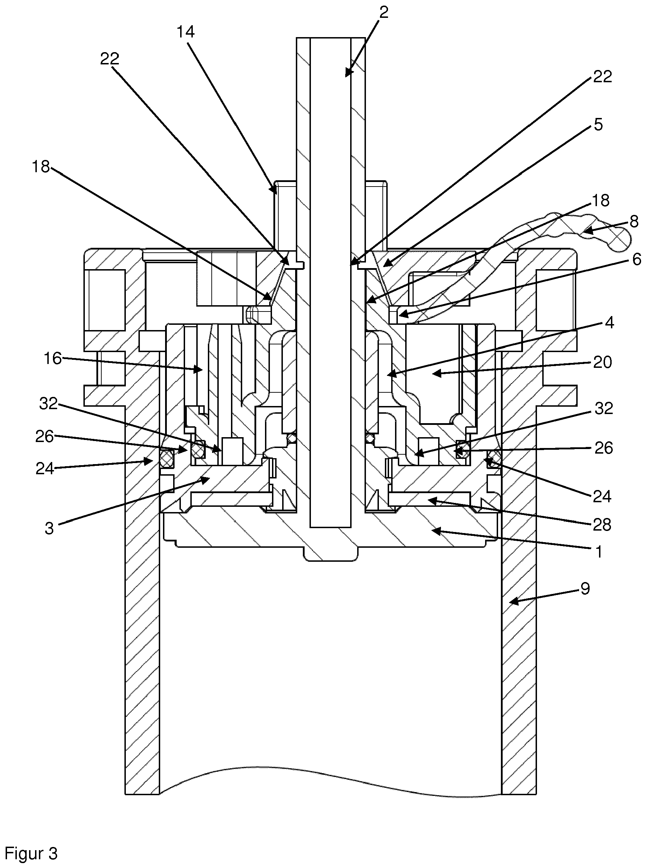

Figur 2 zeigt eine schematische Querschnittansicht durch eine erfindungsgemäße Vorrichtung entlang einer Schnittebene A-A in Figur 1 und Figur 3 zeigt eine schematische Querschnittansicht durch eine erfindungsgemäße Vorrichtung senkrecht zu Figur 2 entlang einer Schnittebene B-B in Figur 1. In den Figuren 2 und 3 ist auch die Kartusche 9 beziehungsweise die Kartuschenwand 9 eingezeichnet, wobei sich der Förderkolben in der Ausgangsposition befindet.

An einer zylinderförmigen Außenwand des Sterilisationskolbens 3 sind zwei Ausnehmungen angeordnet, die sich an der Oberseite des Sterilisationskolbens 3 von etwa der Mitte des Sterilisationskolbens 3 nach Außen aus der Kartusche 9 heraus erstrecken. Zwischen den beiden Schlitzen ist jeweils ein Rastelement 10 an der Außenseite angeordnet, wobei die Breite der Ausnehmungen etwas größer ist, als die Breite der Rastmittel 10. Das bedeutet, die Rastmittel 10 befinden sich auf jeweils einem Streifen 12 der nur an seiner dem Boden des Sterilisationskolbens 3 zugewandten Seite mit der Außenwand des Sterilisationskolbens 3 verbunden ist. Dieser Streifen 12 erstreckt sich bis über den oberen Rand des Sterilisationskolbens 3 und ist als von außen manuell zu betätigendes Griffstück 14 ausgebildet, das im eingebauten Ausgangszustand aus dem Kartuscheninneren herausragt.

In den Kartuscheninnenwänden 9 sind zwei Vertiefungen zur Aufnahme der Rastmittel 10 als Gegenrastmittel angeordnet. Das Griffstück 14 kann in Richtung der Längsachse des Sterilisationskolbens 3 gedrückt werden. Dabei bewegt sich das Rastmittel 10 von der Innenseite der Kartusche 9 weg, aus den Vertiefungen der Kartuscheninnenwand 9 heraus und die Verriegelung wird dadurch gelöst. Theoretisch kann der Sterilisationskolben 3 mit nur einem Rastmittel und einem Griffstück zur Betätigung des Rastmittels aufgebaut werden, ist dann aber weniger leicht bedienbar, weil zwei Griffstücke 14 leicht mit zwei Fingern bedient werden können.

Der Dichtungskolben 4 hat zwei Aussparungen an der Außenseite zur Aufnahme der Griffstücke 14 beziehungsweise der Streifen 12 der Rastmittel 10 des Sterilisationskolbens 3. Die Aussparungen nehmen den Streifen 12 auf, wenn diese zum Lösen der Rastmittel 10 in Richtung des Mischstabs 2 zusammengedrückt werden.

Der Dichtungskolben 4 weist einen Vakuumanschluss 16 auf. Der Dichtungskolben 4 kann erfindungsgemäß besonders bevorzugt ein mit dem Vakuumanschluss 16 verbundenes Rückschlagventil (nicht gezeigt) enthalten. Das Rückschlagventil ist dabei so ausgebildet, das ein Gasstrom nur von der Unterseite des Dichtungskolbens 4 in Richtung des Vakuumanschlusses 16 möglich ist und ein Gasstrom nicht vom Vakuumanschluss 16 in Richtung der Unterseite des Dichtungskolbens 4 fließen kann. Das Rückschlagventil kann als Kugelventil ausgebildet sein. Das Rückschlagventil ist besonders wichtig, wenn das Auspressen des Zementteigs durch Beaufschlagung des Kolbensystems 3, 4, 5 mit Druckgas erfolgen soll, damit kein Druckgas in einen Zementteig im Inneren der Kartusche 9 eindringen kann.

Der Dichtungskolben 4 weist eine Vielzahl kippbarer Klemmbacken 18 auf (in Figur 1 nicht zu sehen), die mit ihrem, dem Boden des Dichtungskolbens 4 abgewandten Ende durch Kippen in Richtung der Längsachse des Mischstabs 2 an den Mischstab 2 pressbar sind. Die Klemmbacken 18 sind durch einen axial geschlitzten Zylinder gebildet, der sich auf der Oberseite des Dichtungskolbens 4 befindet und der die Mischstange 2 umschließt. Dieser Zylinder weist dazu Schlitze auf, die sich vom oberen Rand des Zylinders bis zur Oberseite des Dichtungskolbens 4 erstrecken. Dadurch werden Streifen gebildet, die in Richtung der Mischstange 2 kippbare Klemmbacken 18 darstellen.

Alternativ dazu, aber nicht in den Figuren 1 bis 6 dargestellt, kann auch das Verriegelungselement 5 mindestens eine kippbare Klemmbacke enthalten, die mit ihrem, dem Drehpunkt der Klemmbacke abgewandten Ende durch Kippen in Richtung der Längsachse des Mischstabs 2 an den Mischstab 2 gepresst werden kann. Diese Klemmbacken können durch einen axial geschlitzten Zylinder gebildet sein, der an der Unterseite des Verriegelungselements 5 angeordnet ist, die der Oberseite des Dichtungskolbens 4 zugewandt ist.

Das Verriegelungselement 5 kann mindestens ein elastisch verformbares Element enthalten, das nach Zusammenschieben oder Aufstecken eines Verriegelungselements 5 auf den Dichtungskolben die mindestens eine Klemmbacke 18 an den Mischstab 2 presst. Das Verriegelungselement 5, der Dichtungskolben 4 und der Sterilisationskolben 3 enthalten jeweils eine Aussparung in deren Mittelachse, damit diese auf der Mischstange 2 axial bewegt werden können. Von der Aussparung des Verriegelungselements 5 gehen radial Schlitze aus. Durch die Schlitze werden trapezförmige Streifen gebildet, die bei Verwendung von geeigneten Kunststoffen elastisch in axialer Richtung verformbar sind. Es ist auch möglich, dass das Verriegelungselement 5 nur einen einfachen Kunststoffzylinder zum Anpressen der Klemmbacken enthält. Hierbei wird nur die Elastizität des eingesetzten Kunststoffs genutzt.

Weiterhin kann erfindungsgemäß vorgesehen sein, dass der Dichtungskolben 4 mindestens ein elastisch verformbares Element enthält, das nach Zusammenschieben oder Aufstecken des Verriegelungselements 5 auf den Dichtungskolben 4 die mindestens eine Klemmbacke 18 an den Mischstab 2 presst.

Der Dichtungskolben 4 ist auf den Sterilisationskolben 3 unter Bildung eines zweiteiligen Kolbens aufsteckbar. Nach dem Mischen des Zements wird das Verriegelungselement 5 auf den aus dem Dichtungskolben 4 und dem Sterilisationskolben 3 zusammengesetzten zweiteiligen Kolben unter Bildung eines dreiteiligen Förderkolbens aufgesteckt oder aufgeschoben. Das dreiteilige Kolbensystem umschließt den Mischstab 2.

Eine vorteilhafte Ausgestaltung der Vorrichtung besteht darin, dass zwischen dem Verriegelungselement 5 und dem Dichtungskolben 4 ein manuell entfernbarer Abstandhalter 6 als Sicherungselement 6 angeordnet ist, wodurch dieser ein unbeabsichtigtes Zusammenschieben oder Zusammenstecken des Verriegelungselements 5 und des Dichtungskolbens 4 verhindert.

Dabei kann vorgesehen sein, dass das Verriegelungselement 5 durch Zusammenschieben oder Aufstecken auf den Dichtungskolben 4, formschlüssig oder kraftschlüssig derart mit dem Dichtungskolben 4 verbunden werden kann, dass die mindestens eine Klemmbacke 18 an den Mischstab 2 gepresst wird. Dadurch wird der Mischstab 2 festgelegt und gegen einen vom Vakuum verursachten Zug in Richtung des Kartuscheninneren blockiert.

Das Verriegelungselement 5 ist mit vier Rastelementen 20 unlösbar mit dem Dichtungskolben 4 verbunden. Dadurch kann das Verriegelungselement 5 nicht mehr vom Dichtungskolben 4 gelöst werden, so dass der Mischstab 2 sicher gegen ein Verschieben infolge von der Vakuumeinwirkung gesichert ist.

Der Abstandhalter 6 weist zwei seitliche Aussparungen für die Griffstücke 14 beziehungsweise die Streifen 12 des Sterilisationskolbens 3 auf.

Für die Funktion der Vorrichtung ist es vorteilhaft, dass das dreiteilige Kolbensystem 3, 4, 5 axial in den Mischzylinder beziehungsweise in die Kartusche 9 nach manueller Entriegelung durch Vakuumeinwirkung verschiebbar ist, um einen aus dem Knochenzementpulver und dem Monomer gemischten Knochenzement in Richtung eines Kartuschenkopfs zu bewegen. Dadurch kann der gebildete Knochenzementteig unter weitgehender Vermeidung von Gaseinschlüssen im Vakuum gesammelt werden.

Eine Austragsöffnung der Kartusche 9 besitzt erfindungsgemäß bevorzugt ein Anschlussmittel. Besonders geeignet ist dazu ein Anschlussgewinde. Über das Anschlussgewinde kann ein Austragsrohr angeschraubt werden, mit dem der gemischte Knochenzementteig in Knochenkavitäten, zum Beispiel den proximalen Femur, zur Fixierung von Gelenkendoprothesen eingebracht werden.

Der Mischstab 2 weist gemäß einer besonders bevorzugten Ausgestaltung der Erfindung eine Sollbruchstelle 22 auf, wobei ganz besonders bevorzugt die Solbruchstelle 22 des Mischstabs 2 so angeordnet ist, dass diese durch den Abstandhalter 6 beziehungsweise wegen des Abstandhalters 6 durch das Verriegelungselement 5 verdeckt ist, wenn das Mischelement 1 bis an die Unterseite des Sterilisationskolbens 3 durch den Mischstab 2 gezogen ist. Dadurch kann ein vorzeitiges Abbrechen des Mischstabs 2 weitgehend verhindert werden, so lange sich der Abstandhalter 6 als Sicherungselement zwischen dem Dichtungskolben 4 und dem Verriegelungselement 5 befindet, weil die Solbruchstelle 22 durch das Verriegelungselement 5 abgedeckt ist. Dadurch wird ein fehlerhafter Bedienungsschritt weitgehend verhindert.

Außen um den Sterilisationskolben 3 umlaufend ist eine Dichtung 24 angeordnet, die den Sterilisationskolben 3 mit der Kartuscheninnenwand abdichtet. In gleicher Art und Weise ist außen um den Dichtungskolben 4 eine Dichtung 26 angeordnet, die den Dichtungskolben 4 gegen den Sterilisationskolben 3 abdichtet, wenn dieser eingebaut ist. Die Dichtungen 24, 26 bestehen aus Gummi und sind in einer umlaufenden Nut um den Sterilisationskolben 3 beziehungsweise den Dichtungskolben 4 angeordnet.

Der Sterilisationskolben 3 weist an seiner Unterseite eine gasdurchlässige Porenscheibe 28 auf. Die Porenscheibe 28 wird von einer Verrippung 30 des Sterilisationskolbens 3 gehalten.

Der Vakuumanschluss 16 mündet in einen umlaufenden Gaskanal 32, der in dem Dichtungskolben 4 vorgesehen ist.

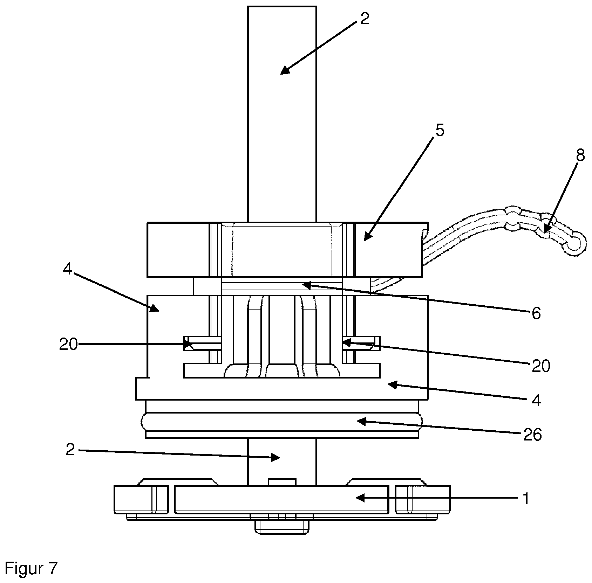

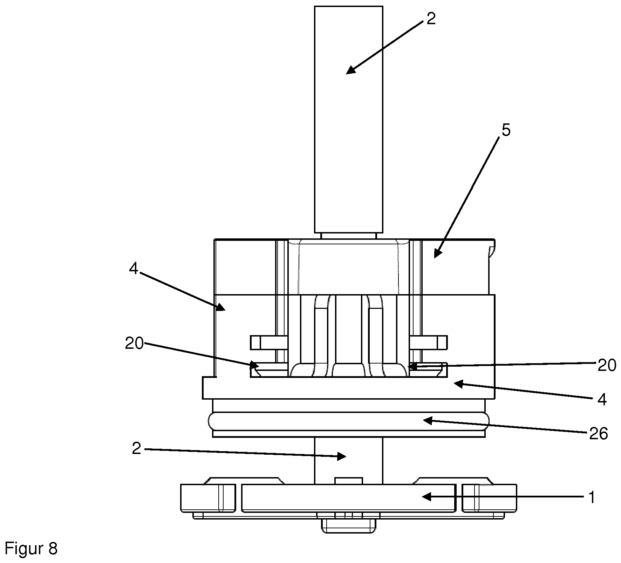

Figur 7 zeigt eine schematische Seitenansicht des Förderkolbens mit Sicherungselement 6 und nicht arretiertem Mischstab 2 und Figur 8 zeigt eine schematische Seitenansicht des Förderkolbens ohne Sicherungselement 6 und mit arretiertem Mischstab 2 zur Verdeutlichung des erfindungsgemäßen Verfahrens und der Funktionsweise erfindungsgemäßer Vorrichtungen.

Es sind in den Figuren 7 und 8 nur zwei Teile des Förderkolbens gezeigt, nämlich der Dichtungskolben 4 und das Verrieglungselement 5. Diese sind wie in dem in den Figuren 1 bis 6 gezeigten Ausführungsbeispiel aufgebaut. Der Sterilisationskolben 3 ist in den Figuren 7 und 8 nicht dargestellt und komplettiert den Förderkolben außen. Mit einer umlaufenden Dichtung 26 ist der Dichtungskolben 4 gegen eine zylindrische Innenwand des Serilisationskolbens (in den Figuren 7 und 8 nicht gezeigt) abgedichtet. Durch eine Durchführung im Inneren des Dichtungskolbens 4 und des Verriegelungselements 5 ist der Mischstab 2 durchgeführt, der das Mischelement 1 hält.

In der in Figur 7 gezeigten Situation ist zwischen dem Dichtungskolben 4 und dem Verriegelungselement 5 noch der Abstandhalter 6 angeordnet. Die Rastelemente 20 des Verriegelungselements 5 greifen in dafür vorgesehene Ausnehmungen im Dichtungskolben 4. Nach der Durchmischung des Kartuscheninhalts mit dem Mischelement 1 wird die Mischstange 2 so weit nach oben gezogen, dass das Mischelement 1 am Sterilisationskolben 3 anliegt. Da der Sterilisationskolben 3 in den Figuren 7 und 8 nicht gezeigt ist, ergibt sich in dieser Position ein Abstand zum Dichtungskolben 4.

Anschließend wird der Abstandhalter 6 am Griff 8 herausgezogen. Dazu hat der Abstandhalter 6 einen Schlitz, in dem der Mischstab 2 angeordnet ist und dessen Breite dem Durchmesser des Mischstabs 2 entspricht oder etwas breiter ist. Wenn der Abstandhalter 6 aus einem elastischen oder leicht verformbaren Material besteht, kann der Schlitz auch bereichsweise enger als der Durchmesser des Mischstabs 2 sein.

Danach wird das Verriegelungselement 5 auf den Dichtungskolben 4 aufgeschoben beziehungsweise aufgesteckt. Dabei wird die Sollbruchstelle 22 des Mischstabs 2 freigelegt. Zudem werden die Klemmbacken 18 auf den Mischstab 2 gepresst und der Mischstab 2 dadurch arretiert. Diese Situation ist in Figur 8 dargestellt. Die Rastmittel 20 des Verrieglungselements 5 rutschen dabei aus den ursprünglichen Ausnehmungen und rutschen in tiefer angeordnete Ausnehmungen in dem Dichtungskolben 4. Die Rastmittel 20 sind an der Außenseite abgeschrägt, so dass eine Bewegung des Verriegelungselements 5 nach unten in den Dichtungskolben 4 möglich ist, ein herausziehen des Verriegelungselements 5 aus dem Dichtungskolben 4 aber nicht möglich ist.

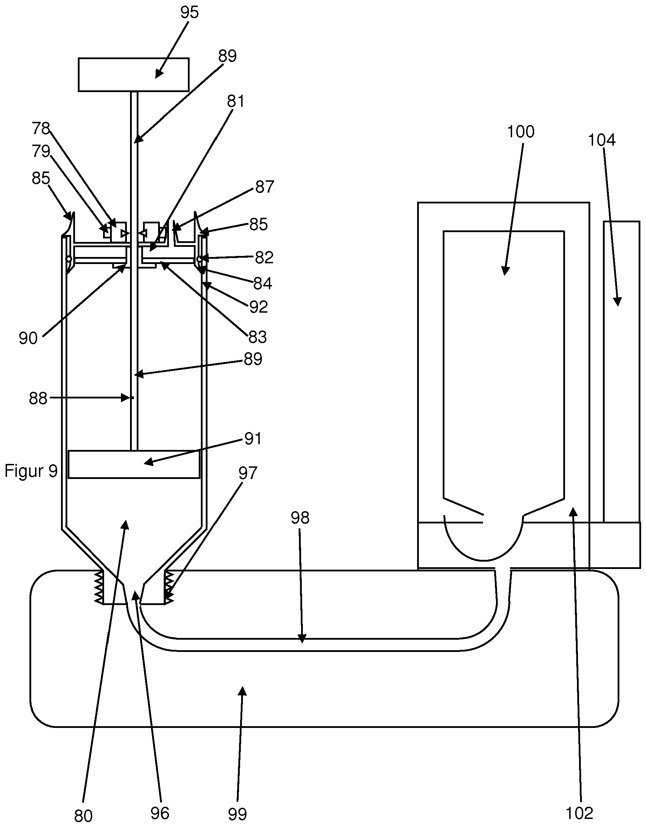

Figur 9 zeigt in schematischer Querschnittansicht den Aufbau eines erfindungsgemäßen Knochenzementsystems mit einer Kartusche 80. In der Kartusche 80 ist ein arretierter Förderkolben 81 angeordnet, der im entarretierten Zustand in Längsrichtung der Kartusche 80 im Inneren der Kartusche 80 beweglich angeordnet ist. Der Förderkolben 81 umfasst eine Dichtung 82 und eine Abstreiflippe 84, die den Förderkolben 81 vollumfänglich umschließen. Zwei Arretierungsvorrichtungen 85 sind auf der Oberseite des Förderkolbens 81 am Förderkolben 81 angeordnet. Auf der Oberseite des Förderkolbens 81 ist ein Vakuumanschluss 87 vorgesehen, über den das Innere der Kartusche 80 evakuierbar ist.

Ein Mischstab 89 mit einer Sollbruchstelle 88 erstreckt sich durch eine Führungshülse 90 durch den Förderkolben 81. Im Inneren der Kartusche 80 ist ein Mischflügel 91 am Mischstab 89 angeordnet. Der Mischstab 89 ist drehbar im Förderkolben 81 gelagert, so dass der Mischflügel 91 in der Kartusche 80 drehbar und in Längsrichtung (in Figur 9 nach oben und unten) verschiebbar ist. Das Innere der Kartusche 80 ist durch eine zylindrische Kartuschenwand 92 begrenzt, so dass im Inneren der Kartusche 80 ein zylindrischer Hohlraum gebildet wird, der, bis auf den unteren trichterförmigen Teil des Innenraums (in Figur 9 im unteren Bereich des Kartuscheninnenraums), das Innere der Kartusche 80 bildet.

Am oberen Ende des Mischstabs 89 ist ein Griff 95 angeordnet, mit dem der Mischstab 89 manuell oder auch über einen Motor bedienbar ist. Auf der dem arretierten Förderkolben 81 gegenüberliegenden Seite der Kartusche 80 mündet der Kartuscheninnenraum in eine Austragsöffnung 96, durch die ein in der Kartusche 80 enthaltenes Material mit dem entarretierten Förderkolben 81 auspressbar ist. Die Kartusche 80 hat im Bereich der Austragsöffnung 96 ein Außengewinde 97, um die Kartusche 80 mit einer Leitung 98 eines Trägers 99 zu verbinden. Dazu wird die Kartusche 80 in ein Innengewinde des Trägers 99 geschraubt.

Auf der anderen Seite des Trägers 99 ist eine Ampulle 100 mit einer Monomerflüssigkeit in einem Behälter 102 angeordnet. Die Ampulle 100 kann über einen Öffnungsmechanismus 104 geöffnet werden, der den Kopf der Ampulle 100 abschert.

Die Kartusche 80 ist zu ca. 2/3 mit einem Knochenzementpulver gefüllt. Zur Sterilisierung des Inhalts wird das Innere der Kartusche 80 zunächst über den Vakuumanschluss 87 evakuiert. Anschließend wird ein sterilisierendes Gas, wie beispielsweise Ethylenoxid in die Kartusche 80 geleitet. Nach einer Zeit, die zum Sterilisieren des Inhalts der Kartusche 80 ausreicht, wird das Ethylenoxid wieder abgepumpt.

Mit Hilfe des Öffnungsmechanismus 104 wird die Ampulle 100 geöffnet und die Monomerflüssigkeit fließt in die Leitung 98. Aufgrund des Unterdrucks im Inneren der Kartusche 80 wird die Monomerflüssigkeit in die Kartusche 80 gezogen und mischt sich dort mit dem Zementpulver. Mit Hilfe des Mischstabs 89 und des Mischflügels 91 können die Monomerflüssigkeit und das Zementpulver durchmischt werden. Aufgrund des Vakuums entstehen in dem entstehenden Zementgemisch keine unerwünschten Lufteinschlüsse. Nach der Durchmischung wird der Mischstab 89 bis zum Anschlag des Mischflügels 91 am Förderkolben 81 nach oben gezogen und mit Hilfe des Verriegelungsmechanismus 79 die Klemmbacken 78 an den Mischstab 89 gepresst. Dabei drücken sich die Dornen der Klemmbacken 78 unterhalb der Sollbruchstelle 88 in den Mischstab 89 und fixieren diesen zusätzlich zu den angepressten Auflageflächen der Klemmbacken 78. Danach kann der Mischstab 89 an der Sollbruchstelle 88 abgebrochen werden, bleibt aber durch die verriegelten Klemmbacken 78 festgehalten.

Anschließend wird der Förderkolben 81 an den Arretierungsvorrichtungen 85 manuell entarretiert. Da im Inneren der Kartusche 80 ein geringerer Druck als in der Umgebung wirkt, wird der Förderkolben 81 inklusive der Klemmbacken 78 und des Verriegelungsmechanismus 79 mit dem Mischstab 89 ins Innere der Kartusche 80 gezogen. Die Kartusche 80 wird an der Vorderseite geöffnet, beispielsweise in dem ein Ventil (nicht gezeigt) bedient wird, oder die Kartusche 80 aus dem Träger 99 herausgeschraubt wird. Durch Eindrücken des Förderkolbens 81 wird dann das Zementgemisch aus dem Inneren des Förderkolbens 81 durch die Austragsöffnung 96 herausgepresst. Der Förderkolben 89 kann auch mit Hilfe eines Druckgases, das auf der Oberseite des Förderkolbens 81 angelegt wird, in die Kartusche 80 befördert werden.

Dazu ist in dem Vakuumanschluss 87 ein Rückschlagventil (nicht gezeigt) angeordnet, das ein Einströmen des Druckgases ins Kartuscheninnere, das heißt, in das Zementgemisch in der Kartusche 80 verhindert.

Auf das Gewinde 97 kann ein Austragsrohr geschraubt werden, so dass der Knochenzement leicht an der gewünschten Stelle appliziert werden kann.

Das erfindungsgemäße Knochenzementsystem mit der zuvor beschriebenen erfindungsgemäßen Vorrichtung zum Mischen und Austragen von Knochenzement, ist also zusammengesetzt aus einem Vorratselement 102 für einen Binder, insbesondere einem Monomer, und einem Basiselement 99, wobei das Basiselement 99 die Vorrichtung mit der Kartusche 80 und das Vorratselement 102 lagert. Dieses Knochenzementsystem stellt mit der erfindungsgemäßen Vorrichtung ein geschlossenes Prepac-System dar, bei dem neben dem Zementpulver auch das Monomer bereits in dem Knochenzementsystem in separaten Kompartimenten vor dem Vermischen der Zementkomponenten enthalten sind, wobei die enthaltene Monomerflüssigkeit erst unmittelbar vor dem Anmischen in das Zementpulver überführt wird.

Das Basiselement 99 enthält dazu das Innengewinde 97 Koppelmittel für eine kraft- und/oder formschlüssige Verbindung mit der Vorrichtung, insbesondere mit einer Austragsöffnung der Vorrichtung.

Das Vorratselement 102 enthält den Behälter 100 für den Binder, insbesondere dem Monomer. Wobei der Behälter 100 bevorzugt ein Glasbehälter ist. Als Glasbehälter kommen besonders Glasampullen in Betracht. Alternativ kann der Behälter 100 als Schlauchbeutel oder als Siegelrandbeutel ausgebildet sein. Diese Beutel können aus diffusionshemmenden Mehrschichtkunststofffolien oder auch aus Aluminiumverbundfolien gefertigt sein.

Das Vorratselement 102 kann erfindungsgemäß ein Ventilmittel (nicht gezeigt) aufweisen, um einen Ausfluss des Monomers aus dem Behälter 100 zu steuern und/oder auszulösen. Dieses Ventilelement kann insbesondere auch als Element 104 zum Öffnen von Glasampullen ausgelegt sein. Daneben kann das Ventilelement auch zum Öffnen von Schlauchbeuteln oder auch Siegelrandbeuteln geeignet sein, wenn Schlauchbeuteln oder auch Siegelrandbeutel als Behälter 100 für das Monomer benutzt werden beziehungsweise benutzbar sein sollen.

Das Basiselement 99 weist ein Leitungsmittel 98 auf, wobei durch das Leitungsmittel 98 das Monomer aus dem Vorratsbehälter 102 in die Vorrichtung, insbesondere in die Kartusche 80 strömt, wenn zuvor mit dem Öffnungsmechanismus beziehungsweise mit dem Ventilmittel 104 der Behälter 100 geöffnet wurde. Besonders vorteilhaft ist dabei, wenn das Monomer durch Einwirkung von Vakuum durch das Leitungsmittel 98 in Kartusche 80 gesaugt wird.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1: Mischelement
- 2: Mischstab
- 3: Sterilisationskolben
- 4: Dichtungskolben
- 5: Verriegelungselement
- 6: Abstandhalter / Sicherungselement
- 8: Griff
- 9: Kartusche
- 10: Rastmittel
- 12: Streifen
- 14: Griffstück
- 16: Vakuumanschluss
- 18: Klemmbacke
- 20: Rastmittel
- 22: Sollbruchstelle
- 24, 26: Dichtung
- 28: Porenscheibe
- 30: Verrippung
- 32: Gaskanal
- 78: Klemmbacke mit Dorn
- 79: Verriegelungselement
- 80: Kartusche
- 81: Förderkolben
- 82: Dichtung
- 83: Porenscheibe
- 84: Abstreiflippe
- 85: Arretierungsvorrichtung
- 87: Vakuumanschluss
- 88: Sollbruchstelle
- 89: Mischstab
- 90: Führungshülse
- 91: Mischelement / Mischflügel
- 92: Kartuschenwand
- 95: Griff
- 96: Austragsöffnung
- 97: Gewinde
- 98: Leitung
- 99: Träger / Basiselelement
- 100: Ampulle
- 102: Behälter / Vorratselement
- 104: Öffnungsmechanismus

## Patentansprüche

1. Vorrichtung zum Mischen und Austragen einer pastösen Masse, insbesondere eines Knochenzements, aufweisend eine an zwei Enden offene oder zu öffnende Kartusche (9, 80), in der ein Mischelement (1, 91) und ein Förderkolben (81) angeordnet sind, wobei der Förderkolben (81) in einer Ausgangsstellung im Bereich eines ersten Kartuschenendes angeordnet ist, das Mischelement (1, 91) an einem Mischstab (2, 89) angeordnet ist, der Mischstab (2, 89) sich durch den Förderkolben (81) in das Kartuscheninnere erstreckt, der Mischstab (2, 89) und der Förderkolben (81) mit der Kartusche (9, 80) eine dichte Verbindung bilden, die das Kartuscheninnere der Vorrichtung nach außen abdichtet, wobei der Förderkolben (81) auf dem Mischstab (2, 89) und in der Kartusche (9, 80) axial bewegbar angeordnet ist, **dadurch gekennzeichnet, dass**
wenigstens eine Klemmbacke (18, 78) am Förderkolben (81) derart angeordnet ist, dass die wenigstens eine Klemmbacke (18, 78) mit einem Verriegelungselement (5, 79) derart an den Mischstab (2, 89) anpressbar ist, dass der Mischstab (2, 89) nicht mehr gegen den Förderkolben (81) verschiebbar ist, wobei das Verriegelungselement (5, 79) ein Teil des Förderkolbens (81) ist, das auf dem Mischstab (2, 89) axial gegen den restlichen Förderkolben (81) verschiebbar ist und das bei einem Zusammenschieben oder Zusammenstecken des Verriegelungselements (5, 79) mit dem restlichen Förderkolben (81) die wenigstens eine Klemmbacke (18, 78) an den Mischstab (2, 89) presst, so dass der Mischstab (2, 89) nicht mehr gegen den Förderkolben (81) verschiebbar ist.

2. Vorrichtung nach Anspruch 1, **dadurch gekennzeichnet, dass**
zwischen dem Verriegelungselement (5, 79) und dem restlichen Förderkolben (81) ein manuell entfernbarer Abstandhalter (6) als Sicherungselement angeordnet ist, der ein unbeabsichtigtes Zusammenschieben oder Zusammenstecken des Verriegelungselements (5, 79) und des restlichen Förderkolbens (81) verhindert.

3. Vorrichtung nach einem der vorangehenden Ansprüche, **dadurch gekennzeichnet, dass**
das Verriegelungselement (5, 79) an dem nach außen weisenden Ende des Förderkolbens (81) angeordnet ist.

4. Vorrichtung nach einem der vorangehenden Ansprüche, **dadurch gekennzeichnet, dass**
der Förderkolben (81) an dem ins Kartuscheninnere weisenden Ende einen Sterilisationskolben (3) aufweist, neben dem das Mischelement (1, 91) in der Kartusche (9, 80) angeordnet ist, wobei der Sterilisationskolben (3) mindestens eine gasdurchlässige Austauschfläche (28, 83) aufweist.

5. Vorrichtung nach Anspruch 4, **dadurch gekennzeichnet, dass**
der Sterilisationskolben (3) mit wenigstens einem lösbaren Rastelement (10) mit der Kartusche (9, 80) verbunden oder verbindbar ist.

6. Vorrichtung nach einem der vorangehenden Ansprüche, **dadurch gekennzeichnet, dass**
der Förderkolben (81) einen Dichtungskolben (4) aufweist, insbesondere als separates Teil des restlichen Förderkolbens (81), wobei der Dichtungskolben (4) auf dem Mischstab (2, 89) axial verschiebbar angeordnet ist und an dem Dichtungskolben (4) die wenigstens eine Klemmbacke (18, 78) angeordnet ist.

7. Vorrichtung nach den Ansprüchen 5 und 6, **dadurch gekennzeichnet, dass**
der Sterilisationskolben (3) mit dem Dichtungskolben (4) und/oder dem Verriegelungselement (5, 79) nach Zusammenschieben oder Zusammenstecken einen zumindest zweiteiligen Förderkolben (81) bildet, vorzugsweise zusammen mit dem Verriegelungselement (5, 79) einen zumindest dreiteiligen Förderkolben (3, 4, 5) bildet.

8. Vorrichtung nach Anspruch 7, **dadurch gekennzeichnet, dass**
der Dichtungskolben (4) auf den Sterilisationskolben (3) aufsteckbar ist und dass das Verriegelungselement (5, 79) auf einen aus dem Dichtungskolben (4) und dem Sterilisationskolben (3) zusammengesetzten Förderkolbenteil (3, 4) unter Bildung eines dreiteiligen Förderkolbens (3, 4, 5) aufsteckbar oder aufschiebbar ist.

9. Vorrichtung nach einem der Ansprüche 5 bis 8, **dadurch gekennzeichnet, dass**
der restliche Förderkolben (81), insbesondere der Dichtungskolben (4) mindestens eine Ausnehmung an der Außenseite zur Aufnahme eines Griffstücks (14) des mindestens einen Rastmittels (10) des Sterilisationskolbens (3) aufweist.

10. Vorrichtung nach einem der vorangehenden Ansprüche, **dadurch gekennzeichnet, dass**
der Förderkolben (81) axial in die Kartusche (9, 80), insbesondere in einen Mischzylinder der Kartusche (9, 80), nach einer Entriegelung durch einen Druck verschiebbar ist, um einen aus Knochenzementpulver und einem Monomer gemischten Knochenzementteig aus dem Kartuscheninneren in Richtung eines Kartuschenkopfs zu bewegen, der an einem, dem ersten Ende der Kartusche (9, 80) gegenüber liegenden zweiten Ende der Kartusche (9, 80) angeordnet ist und der eine zweite Öffnung (96) beinhaltet.

11. Vorrichtung nach einem der vorangehenden Ansprüche, **dadurch gekennzeichnet, dass**
der Mischstab (2, 89) eine Sollbruchstelle (22, 88) aufweist, wobei bevorzugt die Solbruchstelle (22, 88) des Mischstabs (2, 89) derart angeordnet ist, dass die Sollbruchstelle (22, 88) durch einen Abstandhalter (6) verdeckt ist, wenn das Mischelement (1) mit dem Mischstab (2, 89) bis an das ins Kartuscheninnere weisende Ende des Förderkolbens (81) gezogen ist.

12. Vorrichtung nach einem der vorangehenden Ansprüche, **dadurch gekennzeichnet, dass**
der Förderkolben (81), insbesondere der Dichtungskolben (4) und/oder der Sterilisationskolben (3) wenigstens einen Vakuumanschluss (16, 87) aufweist, der eine Verbindung durch den Förderkolben (81) von außen ins Kartuscheninnere bereitstellt, wobei bevorzugt ein Rückschlagventil in dem Vakuumanschluss (16, 87) angeordnet ist.

13. Vorrichtung nach einem der vorangehenden Ansprüche, **dadurch gekennzeichnet, dass**
die zumindest eine Klemmbacke (18, 78) kippbar am Förderkolben (81), insbesondere am Dichtungskolben (4) angeordnet ist und mit ihrem oder ihren dem Boden des Förderkolbens (81) abgewandten Ende oder Enden durch Kippen in Richtung der Längsachse des Mischstabs (2, 89) an den Mischstab (2, 89) pressbar ist oder sind.

14. Vorrichtung nach einem der vorangehenden Ansprüche, **dadurch gekennzeichnet, dass**
das Verriegelungselement (5, 79) mindestens eine kippbare Klemmbacke (18, 78) aufweist, die mit ihrem dem Drehpunkt der Klemmbacke (18, 78) abgewandten Ende durch Kippen in Richtung der Längsachse des Mischstabs (2, 89) an den Mischstab (2, 89) pressbar ist.

15. Vorrichtung nach einem der vorangehenden Ansprüche, **dadurch gekennzeichnet, dass**
das Verriegelungselement (5, 79) und/oder der Dichtungskolben (4) mindestens ein elastisch verformbares Element aufweist, das nach Zusammenschieben oder Aufstecken des Verriegelungselements (5, 79) auf einen restlichen Förderkolben (81) die mindestens eine Klemmbacke (18, 78) an den Mischstab (2, 89) presst.

16. Knochenzementsystem aufweisend eine Vorrichtung nach einem der vorangehenden Ansprüche, ein Vorratselement (102) für einen Binder, insbesondere einem Monomer, und ein Basiselement (99), wobei das Basiselement (99) die Vorrichtung und das Vorratselement (102) lagert und verbindet.

17. Knochenzementsystem nach Anspruch 16, **dadurch gekennzeichnet, dass** das Basiselement (99) ein Koppelmittel (97) für eine kraft- und/oder formschlüssige Verbindung mit der Vorrichtung aufweist, insbesondere eine kraft- und/oder formschlüssige Verbindung mit der Austragsöffnung (96) der Vorrichtung.

18. Knochenzementsystem nach Anspruch 16 oder 17, **dadurch gekennzeichnet, dass** das Vorratselement (102) ein Ventilmittel (104), insbesondere ein manuell bedienbares Ventilmittel (104) aufweist, um einen Ausfluss eines Monomers aus dem Vorratselement (102) zu steuern und/oder auszulösen.

19. Knochenzementsystem nach einem der Ansprüche 16 bis 18, **dadurch gekennzeichnet, dass**
das Basiselement (99) eine Leitung (98) aufweist, wobei die Leitung (98) das Vorratselement (102) mit dem Kartuscheninneren verbindet, so dass ein Monomer aus dem Vorratselement (102) durch die Leitung (98) in die Kartusche (9, 80) strömen kann.

20. Verfahren zum Herstellen eines Knochenzements mit einer Vorrichtung oder einem Knochenzementsystem nach einem der vorangehenden Ansprüche, **gekennzeichnet durch** die chronologischen Verfahrensschritte:
A) Bereitstellen einer mit einem Pulver zumindest bereichsweise gefüllten Kartusche (9, 80);
D) Füllen der Kartusche (9, 80) mit einem Fluid, bevorzugt mit einem Binder, besonders bevorzugt mit einem Monomer;
E) Mischen des Pulvers mit dem Mischelement (1, 91);
F) Verriegeln des Mischstabs (2, 89) im Förderkolben (81) mit der zumindest einen Klemmbacke (18, 78); und
H) Austragen des Zementgemischs durch Vortreiben des Förderkolbens (81).

21. Verfahren nach Anspruch 20, **gekennzeichnet durch**
B) Einfüllen eines sterilisierenden Gases durch eine gasdurchlässige Austauschfläche (28, 83) und Sterilisieren des Kartuscheninnenraums und des Pulvers nach Schritt A) und vor Schritt D) und **dadurch, dass** bei Schritt A) die Kartusche (9, 80) durch einen Sterilisationskolben (3) verschlossen ist.

22. Verfahren nach Anspruch 21, **gekennzeichnet durch**
C) Einstecken eines Dichtungskolbens (4) in den Sterilisationskolben (3) nach Schritt B) und vor Schritt D).

23. Verfahren nach einem der Ansprüche 20 bis 22, **gekennzeichnet durch**
G) Abbrechen des mit der wenigstens einen Klemmbacke (18, 78) verriegelten Mischstabs (2, 89) nach Schritt F) und vor Schritt H), wobei vorzugsweise der Mischstab (2, 89) an einer Sollbruchstelle (22, 88) abgebrochen wird.

24. Verfahren nach einem der Ansprüche 20 bis 23, **dadurch gekennzeichnet, dass**
bei Schritt F) der Mischstab (2, 89) durch ein Aufschieben oder Aufstecken des Verriegelungselements (5, 79) auf den restlichen Förderkolben (81), insbesondere auf den Dichtungskolben (4) verriegelt wird, wobei das Verriegelungselement (5, 79) durch das Aufschieben oder Aufstecken die wenigstens eine Klemmbacke (18, 78) auf den Mischstab (2, 89) presst.

25. Verfahren nach Anspruch 24, **dadurch gekennzeichnet, dass**
bei Schritt F) vor dem Aufschieben oder Aufstecken des Verriegelungselements (5, 79) ein Abstandhalter (6) als Sicherungselement entfernt wird.

26. Verfahren nach einem der Ansprüche 20 bis 25, **dadurch gekennzeichnet, dass**
bei Schritt H) vor dem Austragen des Zementgemischs der Förderkolben (81), insbesondere der mehrteilige Förderkolben (81) durch Lösen wenigstens eines Rastelements (10) von der Kartusche (9, 80) gelöst wird.

## Claims

1. Device for mixing and dispensing a pasty mass, in particular a bone cement, comprising a cartridge (9, 80) that is open or openable on two ends, in which cartridge (9, 80) a mixing element (1, 91) and a feed plunger (81) is arranged, whereby the feed plunger (81) being in a starting position is arranged in the region of a first cartridge end, the mixing element (1, 91) is arranged on a mixing rod (2, 89), the mixing rod (2, 89) extending through the feed plunger (81) into the cartridge inside, the mixing rod (2, 89) and the feed plunger (81) and the cartridge (9, 80) together form a tight connection that seals the cartridge inside of the device with respect to the exterior, whereby the feed plunger (81) is arranged such as to be axially mobile on the mixing rod (2, 89) and in the cartridge (9, 80), **characterized in that** at least one clamping jaw (18, 78) is arranged on the feed plunger (81) in appropriate manner such that the at least one clamping jaw (18, 78) is pressable against the mixing rod (2, 89) by means of a locking element (5, 79), such that the mixing rod (2, 89) is no longer shiftable with respect to the feed plunger (81), wherein the locking element (5, 79) is a part of the feed plunger (81), that is shiftable on the mixing rod (2, 89) in axial direction with respect to the remaining feed plunger (81) and, upon the locking element (5, 79) being slid or plugged together with the remaining feed plunger (81), presses the at least one clamping jaw (18, 78) onto the mixing rod (2, 89) such that the mixing rod (2, 89) is no longer shiftable with respect to the feed plunger (81).

2. Device according to claim 1, **characterised in that**
a manually-removable spacer (6) is arranged as securing element between the locking element (5, 79) and the remaining feed plunger (81) and prevents the locking element (5, 79) and the remaining feed plunger (81) from being slid together or plugged together inadvertently.

3. Device according to any one of the preceding claims, **characterised in that**
the locking element (5, 79) is arranged on the outward-facing end of the feed plunger (81).

4. Device according to any one of the preceding claims, **characterised in that**
the feed plunger (81) comprises, on the end facing the inside of the cartridge, a sterilisation plunger (3) adjacent to which the mixing element (1, 91) is arranged in the cartridge (9, 80), wherein the sterilisation plunger (3) comprises at least one gas-permeable exchange surface (28, 83)

5. Device according to claim 4, **characterised in that**
sterilisation plunger (3) is connected or connectable to the cartridge (9, 80) by means of at least one detachable snap-in element (10).

6. Device according to any one of the preceding claims, **characterised in that**
the feed plunger (81) comprises a sealing plunger (4), in particular as a separate part of the remaining feed plunger (81), whereby the sealing plunger (4) is arranged such that it is shiftable axially on the mixing rod (2, 89) and has at least one clamping jaw (18, 78) arranged on it.

7. Device according to claims 5 and 6, **characterised in that**
the sterilisation plunger (3) and the sealing plunger (4) and/or the locking element (5, 79), slid together or plugged together, form an at least two-part feed plunger (81), preferably together with the locking element (5, 79) form an at least three-part feed plunger (3, 4, 5).

8. Device according to claim 7, **characterised in that**
the sealing plunger (4) is pluggable onto the sterilisation plunger (3) and **in that** the locking element (5, 79) is pluggable or slidable onto a feed plunger part (3, 4) assembled from the sealing plunger (4) and the sterilisation plunger (3), while forming a three-part feed plunger (3, 4, 5).

9. Device according to any one of the claims 5 to 8, **characterised in that**
the remaining feed plunger (81), in particular the sealing plunger (4), comprises at least one recess on the outside for accommodating a grasping part (14) of the at least one snap-in means (10) of the sterilisation plunger (3).

10. Device according to any one of the preceding claims, **characterised in that**
the feed plunger (81) is axially shiftable into the cartridge (9, 80), in particular into a mixing cylinder of the cartridge (9, 80), after an unlocking by means of a pressure in order to move a bone cement dough prepared by mixing bone cement powder and a monomer from the inside of the cartridge towards a cartridge head that is arranged on a second end of the cartridge (9, 80) that is opposite from a first end of the cartridge (9, 80) and includes a second opening (96).

11. Device according to any one of the preceding claims, **characterised in that**
the mixing rod (2, 89) comprises a predetermined breakage site (22, 88), whereby the predetermined breakage site (22, 88) of the mixing rod (2, 89) preferably is arranged appropriately such that the predetermined breakage site (22, 88) is covered by a spacer (6) when the mixing element (1) including the mixing rod (2, 89) is pulled to the end of the feed plunger (81) facing into the inside of the cartridge.

12. Device according to any one of the preceding claims, **characterised in that**
the feed plunger (81), in particular the sealing plunger (4) and/or the sterilisation plunger (3), comprises at least one vacuum connector (16, 87) that provides a connection from outside through the feed plunger (81) into the inside of the cartridge, whereby it is preferred to have a non-return valve arranged in the vacuum connector (16, 87).

13. Device according to any one of the preceding claims, **characterised in that**
the at least one clamping jaw (18, 78) is arranged on the feed plunger (81), in particular on the sealing plunger (4), such as to be tiltable and that it is pressable onto the mixing rod (2, 89), by its end or ends facing away from the bottom of the feed plunger (81) by tilting in the direction of the longitudinal axis of the mixing rod (2, 89).

14. Device according to any one of the preceding claims, **characterised in that**
the locking element (5, 79) comprises at least one tiltable clamping jaw (18, 78) that is pressable onto the mixing rod (2, 89), by its end facing away from the pivot point of the clamping jaw (18, 78) by tilting in the direction of the longitudinal axis of the mixing rod (2, 89).

15. Device according to any one of the preceding claims, **characterised in that**
the locking element (5, 79) and/or the sealing plunger (4) comprise at least one element capable of elastic deformation which presses the at least one clamping jaw (18, 78) onto the mixing rod (2, 89) after the locking element (5, 79) is slid together or plugged onto a remaining feed plunger (81).

16. Bone cement system comprising a device according to any one of the preceding claims, a reservoir element (102) for a binding agent, in particular a monomer, and a base element (99), whereby the base element (99) stores and connects the device and the reservoir element (102).

17. Bone cement system according to claim 16, **characterised in that**
the base element (99) comprises a coupling means (97) for a non-positive-fit-like or positive-fit-like connection to the device, in particular a non-positive-fit-like or positive-fit-like connection to the dispensing opening (96) of the device.

18. Bone cement system according to claim 16 or 17, **characterised in that**
the reservoir element (102) comprises a valve means (104), in particular a valve means for manual operation (104), in order to control and/or trigger an outflow of a monomer from the reservoir element (102).

19. Bone cement system according to any one of the claims 16 to 18, **characterised in that**
the base element (99) comprises a tubing (98), whereby the tubing (98) connects the reservoir element (102) to the inside of the cartridge such that a monomer can flow from the reservoir element (102) through the tubing (98) into the cartridge (9, 80).

20. Method for producing a bone cement using a device or a bone cement system according to any one of the preceding claims, **characterised by** the following chronological steps in the order given:
A) providing a cartridge (9, 80) at least regions of which are filled with a powder;
D) filling the cartridge (9, 80) with a fluid, preferably with a binding agent, particularly preferably with a monomer;
E) mixing the powder by means of the mixing element (1, 91);
F) locking the mixing rod (2, 89) in the feed plunger (81) by means of the at least one clamping jaw (18, 78); and
H) dispensing the cement mixture through propelling the feed plunger (81).

21. Method according to claim 20, **characterised by**
B) filling a sterilising gas through a gas-permeable exchange surface (28, 83) and sterilising the interior space of the cartridge and the powder after step A) and before step D); and by the cartridge (9, 80) being closed by a sterilisation plunger (3) in step A).

22. Method according to claim 21, **characterised by**
C) plugging a sealing plunger (4) into the sterilisation plunger (3) after step B) and before step D).

23. Method according to any one of the claims 20 to 22, **characterised by**
G) breaking-off the mixing rod (2, 89) locked by means of the at least one clamping jaw (18, 78) after step F) and before step H), whereby the mixing rod (2, 89) preferably is broken off at a predetermined breakage site (22, 88).

24. Method according to any one of the claims 20 to 23, **characterised in that**
in step F) the mixing rod (2, 89) is locked by sliding or plugging the locking element (5, 79) onto the remaining feed plunger (81), in particular onto the sealing plunger (4), whereby the locking element (5, 79) presses the at least one clamping jaw (18, 78) onto the mixing rod (2, 89) due to the sliding or plugging.

25. Method according to claim 24, **characterised in that**
a spacer (6) as a securing element removed before the sliding or plugging of the locking element (5, 79) in step F).

26. Method according to any one of the claims 20 to 25, **characterised in that**
the feed plunger (81), in particular the multi-part feed plunger (81), is detached from the cartridge (9, 80) in step H) through detaching at least one snap-in element (10) before dispensing the cement mixture.

## Revendications

1. Dispositif de mélange et d'extraction d'une masse pâteuse, notamment d'un ciment osseux, présentant une cartouche (9, 80) ouverte ou à ouvrir au niveau de deux extrémités, dans laquelle un élément de mélange (1, 91) et un piston de transport (81) sont disposés, où le piston de transport (81) est disposé dans la région de la première extrémité de cartouche dans une position de départ, l'élément de mélange (1, 91) est disposé sur une baguette de mélange (2, 89), la baguette de mélange (2, 89) s'étend à travers le piston de transport (81) dans l'intérieur de la cartouche, la baguette de mélange (2, 89) et le piston de transport (81) forment une liaison étanche avec la cartouche (9, 80) qui rend l'intérieur de la cartouche du dispositif étanche vers l'extérieur, où le piston de transport (81) est disposé sur la baguette de mélange (2, 89) et dans la cartouche (9, 80) en étant mobile axialement, **caractérisé en ce**
**qu'**au moins une mâchoire de serrage (18, 78) est disposée sur le piston de transport (81) de telle manière que l'au moins une mâchoire de serrage (18, 78) peut être pressée contre la baguette de mélange (2, 89) avec un élément de verrouillage (5, 79) de telle manière que la baguette de mélange (2, 89) ne peut plus être déplacée contre le piston de transport (81), où l'élément de verrouillage (5, 79) est une partie du piston de transport (81) qui peut être déplacée axialement sur la baguette de mélange (2, 89) contre le reste du piston de transport (81) et qui presse l'au moins une mâchoire de serrage (18, 78) sur la baguette de mélange (2, 89) lors d'une rassemblement ou d'un assemblage de l'élément de verrouillage (5, 79) avec le reste du piston de transport (81) de sorte que la baguette de mélange (2, 89) ne peut plus de déplacer contre le piston de transport (81).

2. Dispositif selon la revendication 1, **caractérisé en ce**
**qu'**un espaceur (6) pouvant être retiré manuellement servant d'élément de sécurité est disposé entre l'élément de verrouillage (5, 79) et le reste du piston de transport (81), qui empêche un rapprochement ou un assemblage involontaire de l'élément de verrouillage (5, 79) et du reste du piston de transport (81).

3. Dispositif selon l'une des revendications précédentes, **caractérisé en ce que**
l'élément de verrouillage (5, 79) est disposé sur l'extrémité du piston de transport (81) orientée vers l'extérieur.

4. Dispositif selon l'une des revendications précédentes, **caractérisé en ce que**
le piston de transport (81) présente un piston de stérilisation (3) à l'extrémité orientée vers l'intérieur de la cartouche à coté duquel l'élément de mélange (1, 91) est disposé dans la cartouche (9, 80), où le piston de stérilisation (3) présente au moins une surface d'échange (28, 83) perméable aux gaz.

5. Dispositif selon la revendication 4, **caractérisé en ce que**
le piston de stérilisation (3) est relié ou peut être relié avec la cartouche (9, 80) à l'aide d'au moins un élément de blocage (10) amovible.

6. Dispositif selon l'une des revendications précédentes, **caractérisé en ce que**
le piston de transport (81) présente un piston d'étanchéité (4), notamment sous forme d'une partie séparée du reste du piston de transport (81), où le piston d'étanchéité (4) est disposé en pouvant glisser axialement sur la baguette de mélange (2, 89) et l'au moins une mâchoire de serrage (18, 8) est disposée sur le piston d'étanchéité (4).

7. Dispositif selon les revendications 5 et 6, **caractérisé en ce que**
le piston de stérilisation (3), avec le piston d'étanchéité (4), et/ou l'élément de verrouillage (5, 79), forme au moins un piston de transport (81) en deux pièces après le rapprochement ou l'assemblage, forme, de préférence, avec l'élément de verrouillage (5, 79), un au moins piston de transport (3, 4, 5) en trois parties.

8. Dispositif selon la revendication 7, **caractérisé en ce que**
le piston d'étanchéité (4) peut être appliqué sur le piston de stérilisation (3) et que l'élément de verrouillage (5, 79) peut être appliqué sur ou glissé sur une partie de piston de transport (3, 4) assemblé à base du piston d'étanchéité (4) et du piston de stérilisation (3), moyennant la formation d'un piston de transport (3, 4, 5) en trois parties.

9. Dispositif selon l'une des revendications 5 à 8, **caractérisé en ce que**
le reste du piston de transport (81), notamment le piston d'étanchéité (4) présente au moins une cavité sur le côté extérieur pour la réception d'une poignée (14) de l'au moins un moyen de blocage (10) du piston de stérilisation (3).

10. Dispositif selon l'une des revendications précédentes, **caractérisé en ce que**
le piston de transport (81) peut être déplacé axialement dans la cartouche (9, 80), notamment dans un cylindre de mélange de la cartouche (9, 80) après un déverrouillage par une pression afin de déplacer une pâte de ciment osseux à base d'une poudre de ciment osseux et d'un monomère à partir de l'intérieur de la cartouche en direction d'une tête de cartouche qui est disposée à une deuxième extrémité de la cartouche (9, 80) se situant en face de la première extrémité de la cartouche (9, 80) et qui contient un deuxième orifice (96).

11. Dispositif selon l'une des revendications précédentes, **caractérisé en ce que**
la baguette de mélange (2, 89) présente un point de rupture souhaitée (22, 88) où le point de rupture souhaitée (22, 88) de la baguette de mélange (2, 89) est disposé de telle manière que le point de rupture souhaitée (22, 88) est recouvert par un espaceur (6) lorsque l'élément de mélange (1) est tiré jusqu'à l'extrémité du piston de transport (81) orientée vers l'intérieur de la cartouche.

12. Dispositif selon l'une des revendications précédentes, **caractérisé en ce que**
le piston de transport (81), notamment le piston d'étanchéité (4), et/ou le piston de stérilisation (3), présente au moins un raccord au vide (16, 87) qui met à disposition une liaison par le piston de transport (81) à partir de l'extérieur vers l'intérieur de la cartouche, où de préférence un clapet anti-retour est disposé dans le raccord au vide (16, 87).

13. Dispositif selon l'une des revendications précédentes, **caractérisé en ce que**
l'au moins une mâchoire de serrage (18, 78) est disposée en pouvant basculer sur le piston de transport (81), notamment sur le piston d'étanchéité (4), et qui peut être pressée ou est pressée sur la baguette de mélange (2, 89) avec son extrémité ou son extrémité opposée au fond du piston de transport (81) ou ses extrémités par un basculement en direction de l'axe longitudinal de la baguette de mélange (2, 89).

14. Dispositif selon l'une des revendications précédentes, **caractérisé en ce que**
l'élément de verrouillage (5, 79) présente au moins une mâchoire de serrage (18, 78) pouvant être basculée qui peut être pressée avec son extrémité opposée au point de rotation de la mâchoire de serrage (18, 78), par un basculement en direction de l'axe longitudinal de la baguette de mélange (2, 89) sur la baguette de mélange (2, 89).

15. Dispositif selon l'une des revendications précédentes, **caractérisé en ce que**
l'élément de verrouillage (5, 79), et/ou le piston d'étanchéité (4), présente au moins un élément élastiquement déformable qui presse l'au moins une mâchoire de serrage (18, 78) sur la baguette de mélange (2, 89) après le rapprochement ou l'assemblage de l'élément de verrouillage (5, 79) sur le reste du piston de transport (81).

16. Système de ciment osseux présentant un dispositif selon l'une des revendications précédentes, un élément de réserve (102) pour un liant, notamment un monomère, et un élément de base (99), où l'élément de base (99) stocke et relie le dispositif et l'élément de réserve (102).

17. Système de ciment osseux selon la revendication 16, **caractérisé en ce que** l'élément de base (99) présente un moyen de couplage pour une liaison par complémentarité des matières et/ou des formes avec l'orifice de sortie (96) du dispositif.

18. Système de ciment osseux selon la revendication 16 ou la revendication 17, **caractérisé en ce que**
l'élément de réserve (102) présente un moyen de mise à l'air (104), notamment un moyen de mise à l'air (104) actionnable manuellement afin de commander et/ou de déclencher l'écoulement d'un monomère à partir de l'élément de réserve (102).

19. Système de ciment osseux selon l'une des revendications 16 à 18, **caractérisé en ce que**
l'élément de base (99) présente une conduite (98), où la conduite (98) relie l'élément de réserve (102) avec l'intérieur de la cartouche de sorte qu'un monomère peut s'écouler hors de l'élément de réserve (102) à travers la conduite (98) dans la cartouche (9, 80).

20. Procédé de fabrication d'un ciment osseux avec un dispositif ou un système de ciment osseux selon l'une des revendications précédentes, **caractérisé par** les étapes de procédé chronologiques :
A) de fourniture d'une cartouche (9, 80) remplie au moins partiellement avec une poudre ;
D) de remplissage de la cartouche (9, 80) avec un fluide, de préférence avec un liant, de manière particulièrement préférée, avec un monomère ;
E) de mélange de la poudre avec l'élément de mélange (1, 91) ;
F) de verrouillage de la baguette de mélange (2, 89) sur le piston de transport (81) avec l'au moins une mâchoire de serrage (18, 78) ; et
H) d'évacuation du mélange de ciment par une avancée du piston de transport (81).

21. Procédé selon la revendication 20, **caractérisé par**
B) un remplissage d'un gaz de stérilisation par une surface d'échange (28, 83) perméable aux gaz et une stérilisation de l'espace intérieur de la cartouche et de la poudre après l'étape A) et avant l'étape D), et en ce que lors de l'étape A), la cartouche (9, 80) est fermée par un piston de stérilisation (3).

22. Procédé selon la revendication 21, **caractérisé par**
C) la mise en place d'un piston d'étanchéité (4) dans le piston de stérilisation (3) après l'étape B) et avant l'étape D).

23. Procédé selon l'une des revendications 20 à 22, **caractérisé par**
G) une cassure de la baguette de mélange (2, 89) verrouillée avec l'au moins une mâchoire de serrage (18, 78) après l'étape F) et avant l'étape H), où la baguette de mélange (2, 89) est cassée de préférence au niveau du point de rupture souhaitée (22, 88).

24. Procédé selon l'une des revendications 20 à 23, **caractérisé en ce que**
lors de l'étape F), la baguette de mélange (2, 89) est verrouillée sur le reste du piston de transport (81), notamment sur le piston d'étanchéité (4) par un emmanchement ou un assemblage de l'élément de verrouillage (5, 79) sur le reste du piston de transport (81), où l'élément de verrouillage (5, 79) presse l'au moins une mâchoire de serrage (18, 78) sur la baguette de mélange (2, 89) par l'emmanchement ou l'assemblage.

25. Procédé selon la revendication 24, **caractérisé en ce que**
lors de l'étape F), avant l'emmanchement ou l'assemblage de l'élément de verrouillage (5, 79) un espaceur (6) est enlevé en tant qu'élément de sécurité.

26. Procédé selon l'une des revendications 20 à 25, **caractérisé en ce que**
lors de l'étape H), avant l'évacuation du mélange de ciment, le piston de transport (81), notamment le piston de transport (81) en plusieurs parties, est détaché de la cartouche (9, 80) par le détachement d'au moins un élément de blocage (10).
